(19) Europäisches Patentamt
European Patent Office
Office européen des brevets

(11) EP 1 795 592 A2

(12) **EUROPEAN PATENT APPLICATION**

(43) Date of publication:
**13.06.2007 Bulletin 2007/24**

(51) Int Cl.:
***C12N 15/06*** *(2006.01)* ***C07K 16/18*** *(2006.01)*

(21) Application number: **07005887.0**

(22) Date of filing: **04.09.2003**

(84) Designated Contracting States:
**AT BE BG CH CY CZ DE DK EE ES FI FR GB GR HU IE IT LI LU MC NL PT RO SE SI SK TR**

(30) Priority: **04.09.2002 WOPCT/JP02/08999**

(62) Document number(s) of the earlier application(s) in accordance with Art. 76 EPC:
**03794236.4 / 1 541 680**

(71) Applicant: **CHUGAI SEIYAKU KABUSHIKI KAISHA**
**Tokyo, 115-8543 (JP)**

(72) Inventors:
- **Aburatani, H.**
  **Meguro-ku,**
  **Tokyo 153-8 (JP)**
- **Midorikawa, Y.**
  **Meguro-ku,**
  **Tokyo 153-8 (JP)**
- **Nakano, Kiyotaka**
  **Gotenba-shi,**
  **Shizuoka 412-8513 (JP)**
- **Ohizumi, Iwao**
  **Gotenba-shi,**
  **Shizuoka 412-8513 (JP)**
- **Ito, Yukio**
  **Meguro-ku**
  **Tokyo 153-0041 (JP)**
- **Tokita, Susumu**
  **Meguro-ku**
  **Tokyo 153-0041 (JP)**

(74) Representative: **Woods, Geoffrey Corlett**
**J.A. KEMP & CO.**
**14 South Square**
**Gray's Inn**
**London WC1R 5JJ (GB)**

Remarks:
This application was filed on 22 - 03 - 2007 as a divisional application to the application mentioned under INID code 62.

(54) **Antibody against secreted N-terminal peptide of GPC3 present in blood or C-terminal peptide of GPC3**

(57) Disclosed is an antibody against a secreted form of GPC3 capable of detecting a secreted form of glypican 3 (GPC3) in a test sample. It is possible to determine whether a subject suffers from cancer, in particular hepatoma. Also disclosed is an antibody against GPC as well as a cell disrupting agent and an anti-cancer agent comprising the same, which can disrupt cells, in particular cancer cells.

EP 1 795 592 A2

SEQUENCE LISTING

```
<110> CHUGAI SEIYAKU KABUSHIKI KAISHA

<120> ANTIBODY AGAINST SECRETED N-TERMINAL PEPTIDE OF GPC3 PRESENT IN BLOOD
OR C-TERMINAL PEPTIDE OF GPC3

<130> N.94176B GCW

<150> EP 03794236.4
<151> 2003-09-04

<150> PCT/JP03/11318
<151> 2003-09-04

<150> PCT/JP02/08999
<151> 2002-09-04

<160> 24

<170> PatentIn Ver. 2.1

<210> 1
<211> 31
<212> DNA
<213> Artificial Sequence

<220>
<223> Description of Artificial Sequence: Synthetic DNA

<400> 1
gatatcatgg ccgggaccgt gcgcaccgcg t                                 31
```

<210> 2
<211> 31
<212> DNA
<213> Artificial Sequence

<220>
<223> Description of Artificial Sequence: Synthetic DNA

<400> 2
gctagctcag tgcaccagga agaagaagca c 31

<210> 3
<211> 2300
<212> DNA
<213> Homo sapiens

<220>
<221> CDS
<222> (109)..(1851)

<400> 3
cagcacgtct cttgctcctc agggccactg ccaggcttgc cgagtcctgg gactgctctc 60

gctccggctg ccactctccc gcgctctcct agctccctgc gaagcagg atg gcc ggg 117
Met Ala Gly
1

acc gtg cgc acc gcg tgc ttg gtg gtg gcg atg ctg ctc agc ttg gac 165
Thr Val Arg Thr Ala Cys Leu Val Val Ala Met Leu Leu Ser Leu Asp
5 10 15

ttc ccg gga cag gcg cag ccc ccg ccg ccg ccg ccg gac gcc acc tgt 213
Phe Pro Gly Gln Ala Gln Pro Pro Pro Pro Pro Pro Asp Ala Thr Cys
20 25 30 35

cac caa gtc cgc tcc ttc ttc cag aga ctg cag ccc gga ctc aag tgg 261
His Gln Val Arg Ser Phe Phe Gln Arg Leu Gln Pro Gly Leu Lys Trp
40 45 50

gtg cca gaa act ccc gtg cca gga tca gat ttg caa gta tgt ctc cct 309
Val Pro Glu Thr Pro Val Pro Gly Ser Asp Leu Gln Val Cys Leu Pro
55 60 65

aag ggc cca aca tgc tgc tca aga aag atg gaa gaa aaa tac caa cta 357
Lys Gly Pro Thr Cys Cys Ser Arg Lys Met Glu Glu Lys Tyr Gln Leu
70 75 80

aca gca cga ttg aac atg gaa cag ctg ctt cag tct gca agt atg gag 405
Thr Ala Arg Leu Asn Met Glu Gln Leu Leu Gln Ser Ala Ser Met Glu
85 90 95

ctc aag ttc tta att att cag aat gct gcg gtt ttc caa gag gcc ttt 453
Leu Lys Phe Leu Ile Ile Gln Asn Ala Ala Val Phe Gln Glu Ala Phe
100 105 110 115

gaa att gtt gtt cgc cat gcc aag aac tac acc aat gcc atg ttc aag 501

Glu Ile Val Val Arg His Ala Lys Asn Tyr Thr Asn Ala Met Phe Lys
120 125 130

aac aac tac cca agc ctg act cca caa gct ttt gag ttt gtg ggt gaa 549
Asn Asn Tyr Pro Ser Leu Thr Pro Gln Ala Phe Glu Phe Val Gly Glu
135 140 145

ttt ttc aca gat gtg tct ctc tac atc ttg ggt tct gac atc aat gta 597
Phe Phe Thr Asp Val Ser Leu Tyr Ile Leu Gly Ser Asp Ile Asn Val
150 155 160

gat gac atg gtc aat gaa ttg ttt gac agc ctg ttt cca gtc atc tat 645
Asp Asp Met Val Asn Glu Leu Phe Asp Ser Leu Phe Pro Val Ile Tyr
165 170 175

acc cag cta atg aac cca ggc ctg cct gat tca gcc ttg gac atc aat 693
Thr Gln Leu Met Asn Pro Gly Leu Pro Asp Ser Ala Leu Asp Ile Asn
180 185 190 195

gag tgc ctc cga gga gca aga cgt gac ctg aaa gta ttt ggg aat ttc 741
Glu Cys Leu Arg Gly Ala Arg Arg Asp Leu Lys Val Phe Gly Asn Phe
200 205 210

ccc aag ctt att atg acc cag gtt tcc aag tca ctg caa gtc act agg 789
Pro Lys Leu Ile Met Thr Gln Val Ser Lys Ser Leu Gln Val Thr Arg
215 220 225

atc ttc ctt cag gct ctg aat ctt gga att gaa gtg atc aac aca act 837
Ile Phe Leu Gln Ala Leu Asn Leu Gly Ile Glu Val Ile Asn Thr Thr

230 235 240

gat cac ctg aag ttc agt aag gac tgt ggc cga atg ctc acc aga atg 885
Asp His Leu Lys Phe Ser Lys Asp Cys Gly Arg Met Leu Thr Arg Met
245 250 255

tgg tac tgc tct tac tgc cag gga ctg atg atg gtt aaa ccc tgt ggc 933
Trp Tyr Cys Ser Tyr Cys Gln Gly Leu Met Met Val Lys Pro Cys Gly
260 265 270 275

ggt tac tgc aat gtg gtc atg caa ggc tgt atg gca ggt gtg gtg gag 981
Gly Tyr Cys Asn Val Val Met Gln Gly Cys Met Ala Gly Val Val Glu
280 285 290

att gac aag tac tgg aga gaa tac att ctg tcc ctt gaa gaa ctt gtg 1029
Ile Asp Lys Tyr Trp Arg Glu Tyr Ile Leu Ser Leu Glu Glu Leu Val
295 300 305

aat ggc atg tac aga atc tat gac atg gag aac gta ctg ctt ggt ctc 1077
Asn Gly Met Tyr Arg Ile Tyr Asp Met Glu Asn Val Leu Leu Gly Leu
310 315 320

ttt tca aca atc cat gat tct atc cag tat gtc cag aag aat gca gga 1125
Phe Ser Thr Ile His Asp Ser Ile Gln Tyr Val Gln Lys Asn Ala Gly
325 330 335

aag ctg acc acc act att ggc aag tta tgt gcc cat tct caa caa cgc 1173
Lys Leu Thr Thr Thr Ile Gly Lys Leu Cys Ala His Ser Gln Gln Arg
340 345 350 355

caa tat aga tct gct tat tat cct gaa gat ctc ttt att gac aag aaa 1221
Gln Tyr Arg Ser Ala Tyr Tyr Pro Glu Asp Leu Phe Ile Asp Lys Lys
360 365 370

gta tta aaa gtt gct cat gta gaa cat gaa gaa acc tta tcc agc cga 1269
Val Leu Lys Val Ala His Val Glu His Glu Glu Thr Leu Ser Ser Arg
375 380 385

aga agg gaa cta att cag aag ttg aag tct ttc atc agc ttc tat agt 1317
Arg Arg Glu Leu Ile Gln Lys Leu Lys Ser Phe Ile Ser Phe Tyr Ser
390 395 400

gct ttg cct ggc tac atc tgc agc cat agc cct gtg gcg gaa aac gac 1365
Ala Leu Pro Gly Tyr Ile Cys Ser His Ser Pro Val Ala Glu Asn Asp
405 410 415

acc ctt tgc tgg aat gga caa gaa ctc gtg gag aga tac agc caa aag 1413
Thr Leu Cys Trp Asn Gly Gln Glu Leu Val Glu Arg Tyr Ser Gln Lys
420 425 430 435

gca gca agg aat gga atg aaa aac cag ttc aat ctc cat gag ctg aaa 1461
Ala Ala Arg Asn Gly Met Lys Asn Gln Phe Asn Leu His Glu Leu Lys
440 445 450

atg aag ggc cct gag cca gtg gtc agt caa att att gac aaa ctg aag 1509
Met Lys Gly Pro Glu Pro Val Val Ser Gln Ile Ile Asp Lys Leu Lys
455 460 465

cac att aac cag ctc ctg aga acc atg tct atg ccc aaa ggt aga gtt 1557
His Ile Asn Gln Leu Leu Arg Thr Met Ser Met Pro Lys Gly Arg Val
470 475 480

ctg gat aaa aac ctg gat gag gaa ggg ttt gaa agt gga gac tgc ggt 1605
Leu Asp Lys Asn Leu Asp Glu Glu Gly Phe Glu Ser Gly Asp Cys Gly
485 490 495

gat gat gaa gat gag tgc att gga ggc tct ggt gat gga atg ata aaa 1653
Asp Asp Glu Asp Glu Cys Ile Gly Gly Ser Gly Asp Gly Met Ile Lys
500 505 510 515

gtg aag aat cag ctc cgc ttc ctt gca gaa ctg gcc tat gat ctg gat 1701
Val Lys Asn Gln Leu Arg Phe Leu Ala Glu Leu Ala Tyr Asp Leu Asp
520 525 530

gtg gat gat gcg cct gga aac agt cag cag gca act ccg aag gac aac 1749
Val Asp Asp Ala Pro Gly Asn Ser Gln Gln Ala Thr Pro Lys Asp Asn
535 540 545

gag ata agc acc ttt cac aac ctc ggg aac gtt cat tcc ccg ctg aag 1797
Glu Ile Ser Thr Phe His Asn Leu Gly Asn Val His Ser Pro Leu Lys
550 555 560

ctt ctc acc agc atg gcc atc tcg gtg gtg tgc ttc ttc ttc ctg gtg 1845
Leu Leu Thr Ser Met Ala Ile Ser Val Val Cys Phe Phe Phe Leu Val
565 570 575

cac tga ctgcctggtg cccagcacat gtgctgccct acagcaccct gtggtcttcc 1901

His
580

tcgataaagg gaaccacttt cttatttttt tctatttttt tttttttgtt atcctgtata 1961

cctcctccag ccatgaagta gaggactaac catgtgttat gttttcgaaa atcaaatggt 2021

atcttttgga ggaagataca ttttagtggt agcatataga ttgtcctttt gcaaagaaag 2081

aaaaaaaacc atcaagttgt gccaaattat tctcctatgt ttggctgcta gaacatggtt 2141

accatgtctt tctctctcac tccctccctt tctatcgttc tctctttgca tggatttctt 2201

tgaaaaaaaa taaattgctc aaataaaaaa aaaaaaaaaa aaaaaaaaaa aaaaaaaaaa 2261

aaaaaaaaaa aaaaaaaaaa aaaaaaaaaa aaaaaaaaa 2300

<210> 4
<211> 580
<212> PRT
<213> Homo sapiens

<400> 4
Met Ala Gly Thr Val Arg Thr Ala Cys Leu Val Val Ala Met Leu Leu
1 5 10 15
Ser Leu Asp Phe Pro Gly Gln Ala Gln Pro Pro Pro Pro Pro Pro Asp
20 25 30
Ala Thr Cys His Gln Val Arg Ser Phe Phe Gln Arg Leu Gln Pro Gly

35 40 45

Leu Lys Trp Val Pro Glu Thr Pro Val Pro Gly Ser Asp Leu Gln Val

50 55 60

Cys Leu Pro Lys Gly Pro Thr Cys Cys Ser Arg Lys Met Glu Glu Lys

65 70 75 80

Tyr Gln Leu Thr Ala Arg Leu Asn Met Glu Gln Leu Leu Gln Ser Ala

85 90 95

Ser Met Glu Leu Lys Phe Leu Ile Ile Gln Asn Ala Ala Val Phe Gln

100 105 110

Glu Ala Phe Glu Ile Val Val Arg His Ala Lys Asn Tyr Thr Asn Ala

115 120 125

Met Phe Lys Asn Asn Tyr Pro Ser Leu Thr Pro Gln Ala Phe Glu Phe

130 135 140

Val Gly Glu Phe Phe Thr Asp Val Ser Leu Tyr Ile Leu Gly Ser Asp

145 150 155 160

Ile Asn Val Asp Asp Met Val Asn Glu Leu Phe Asp Ser Leu Phe Pro

165 170 175

Val Ile Tyr Thr Gln Leu Met Asn Pro Gly Leu Pro Asp Ser Ala Leu

180 185 190

Asp Ile Asn Glu Cys Leu Arg Gly Ala Arg Arg Asp Leu Lys Val Phe

195 200 205

Gly Asn Phe Pro Lys Leu Ile Met Thr Gln Val Ser Lys Ser Leu Gln

210 215 220

Val Thr Arg Ile Phe Leu Gln Ala Leu Asn Leu Gly Ile Glu Val Ile

225 230 235 240

Asn Thr Thr Asp His Leu Lys Phe Ser Lys Asp Cys Gly Arg Met Leu

245 250 255

Thr Arg Met Trp Tyr Cys Ser Tyr Cys Gln Gly Leu Met Met Val Lys

260 265 270

Pro Cys Gly Gly Tyr Cys Asn Val Val Met Gln Gly Cys Met Ala Gly
275 280 285

Val Val Glu Ile Asp Lys Tyr Trp Arg Glu Tyr Ile Leu Ser Leu Glu
290 295 300

Glu Leu Val Asn Gly Met Tyr Arg Ile Tyr Asp Met Glu Asn Val Leu
305 310 315 320

Leu Gly Leu Phe Ser Thr Ile His Asp Ser Ile Gln Tyr Val Gln Lys
325 330 335

Asn Ala Gly Lys Leu Thr Thr Thr Ile Gly Lys Leu Cys Ala His Ser
340 345 350

Gln Gln Arg Gln Tyr Arg Ser Ala Tyr Tyr Pro Glu Asp Leu Phe Ile
355 360 365

Asp Lys Lys Val Leu Lys Val Ala His Val Glu His Glu Glu Thr Leu
370 375 380

Ser Ser Arg Arg Arg Glu Leu Ile Gln Lys Leu Lys Ser Phe Ile Ser
385 390 395 400

Phe Tyr Ser Ala Leu Pro Gly Tyr Ile Cys Ser His Ser Pro Val Ala
405 410 415

Glu Asn Asp Thr Leu Cys Trp Asn Gly Gln Glu Leu Val Glu Arg Tyr
420 425 430

Ser Gln Lys Ala Ala Arg Asn Gly Met Lys Asn Gln Phe Asn Leu His
435 440 445

Glu Leu Lys Met Lys Gly Pro Glu Pro Val Val Ser Gln Ile Ile Asp
450 455 460

Lys Leu Lys His Ile Asn Gln Leu Leu Arg Thr Met Ser Met Pro Lys
465 470 475 480

Gly Arg Val Leu Asp Lys Asn Leu Asp Glu Glu Gly Phe Glu Ser Gly
485 490 495

Asp Cys Gly Asp Asp Glu Asp Glu Cys Ile Gly Gly Ser Gly Asp Gly

500 505 510
Met Ile Lys Val Lys Asn Gln Leu Arg Phe Leu Ala Glu Leu Ala Tyr
515 520 525
Asp Leu Asp Val Asp Asp Ala Pro Gly Asn Ser Gln Gln Ala Thr Pro
530 535 540
Lys Asp Asn Glu Ile Ser Thr Phe His Asn Leu Gly Asn Val His Ser
545 550 555 560
Pro Leu Lys Leu Leu Thr Ser Met Ala Ile Ser Val Val Cys Phe Phe
565 570 575
Phe Leu Val His
580

<210> 5
<211> 31
<212> DNA
<213> Artificial Sequence

<220>
<223> Description of Artificial Sequence: Synthetic DNA

<400> 5
atagaattcc accatggccg ggaccgtgcg c 31

<210> 6
<211> 31
<212> DNA

<213> Artificial Sequence

<220>
<223> Description of Artificial Sequence: Synthetic DNA

<400> 6
ataggatccc ttcagcgggg aatgaacgtt c 31

<210> 7
<211> 21
<212> DNA
<213> Artificial Sequence

<220>
<223> Description of Artificial Sequence: Synthetic DNA

<400> 7
gggccagtgg atagacagat g 21

<210> 8
<211> 23
<212> DNA
<213> Artificial Sequence

<220>
<223> Description of Artificial Sequence: Synthetic DNA

<400> 8
gctcactgga tggtgggaag atg 23

<210> 9
<211> 1392
<212> DNA
<213> Artificial Sequence

<220>
<221> CDS
<222> (1)..(1389)

<220>
<223> Description of Artificial Sequence: Mouse-human chimeric antibody (M3C11 H chain)

<400> 9
atg aac ttc ggg ctc acc ttg att ttc ctt gtc ctt act tta aaa ggt 48
Met Asn Phe Gly Leu Thr Leu Ile Phe Leu Val Leu Thr Leu Lys Gly
1 5 10 15

gtc cag tgt gag gtg caa ctg gtg gag tct ggg gga ggc tta gtg aag 96
Val Gln Cys Glu Val Gln Leu Val Glu Ser Gly Gly Gly Leu Val Lys
20 25 30

cct gga gga tcc ctg aaa ctc tcc tgt gca gcc tct gga ttc act ttc 144
Pro Gly Gly Ser Leu Lys Leu Ser Cys Ala Ala Ser Gly Phe Thr Phe
35 40 45

agt cgc tat gcc atg tct tgg gtt cgc cag att cca gag aag ata ctg 192
Ser Arg Tyr Ala Met Ser Trp Val Arg Gln Ile Pro Glu Lys Ile Leu
50 55 60

gag tgg gtc gca gcc att gat agt agt ggt ggt gac acc tac tat tta 240
Glu Trp Val Ala Ala Ile Asp Ser Ser Gly Gly Asp Thr Tyr Tyr Leu
65 70 75 80

gac act gtg aag gac cga ttc acc atc tcc aga gac aat gcc aat aat 288
Asp Thr Val Lys Asp Arg Phe Thr Ile Ser Arg Asp Asn Ala Asn Asn
85 90 95

acc ctg cac ctg caa atg cgc agt ctg agg tct gag gac aca gcc ttg 336
Thr Leu His Leu Gln Met Arg Ser Leu Arg Ser Glu Asp Thr Ala Leu
100 105 110

tat tac tgt gta aga cag ggg ggg gct tac tgg ggc caa ggg.act ctg 384
Tyr Tyr Cys Val Arg Gln Gly Gly Ala Tyr Trp Gly Gln Gly Thr Leu
115 120 125

gtc act gtc tct gca gct agc acc aag ggc cca tcg gtc ttc ccc ctg 432
Val Thr Val Ser Ala Ala Ser Thr Lys Gly Pro Ser Val Phe Pro Leu
130 135 140

gca ccc tcc tcc aag agc acc tct ggg ggc aca gcg gcc ctg ggc tgc 480
Ala Pro Ser Ser Lys Ser Thr Ser Gly Gly Thr Ala Ala Leu Gly Cys
145 150 155 160

ctg gtc aag gac tac ttc ccc gaa ccg gtg acg gtg tcg tgg aac tca 528
Leu Val Lys Asp Tyr Phe Pro Glu Pro Val Thr Val Ser Trp Asn Ser
165 170 175

ggc gcc ctg acc agc ggc gtg cac acc ttc ccg gct gtc cta cag tcc 576
Gly Ala Leu Thr Ser Gly Val His Thr Phe Pro Ala Val Leu Gln Ser
180 185 190

tca gga ctc tac tcc ctc agc agc gtg gtg acc gtg ccc tcc agc agc 624
Ser Gly Leu Tyr Ser Leu Ser Ser Val Val Thr Val Pro Ser Ser Ser
195 200 205

ttg ggc acc cag acc tac atc tgc aac gtg aat cac aag ccc agc aac 672
Leu Gly Thr Gln Thr Tyr Ile Cys Asn Val Asn His Lys Pro Ser Asn
210 215 220

acc aag gtg gac aag aaa gtt gag ccc aaa tct tgt gac aaa act cac 720
Thr Lys Val Asp Lys Lys Val Glu Pro Lys Ser Cys Asp Lys Thr His
225 230 235 240

aca tgc cca ccg tgc cca gca cct gaa ctc ctg ggg gga ccg tca gtc 768
Thr Cys Pro Pro Cys Pro Ala Pro Glu Leu Leu Gly Gly Pro Ser Val
245 250 255

ttc ctc ttc ccc cca aaa ccc aag gac acc ctc atg atc tcc cgg acc 816
Phe Leu Phe Pro Pro Lys Pro Lys Asp Thr Leu Met Ile Ser Arg Thr
260 265 270

cct gag gtc aca tgc gtg gtg gtg gac gtg agc cac gaa gac cct gag 864

Pro Glu Val Thr Cys Val Val Val Asp Val Ser His Glu Asp Pro Glu
275 280 285

gtc aag ttc aac tgg tac gtg gac ggc gtg gag gtg cat aat gcc aag 912
Val Lys Phe Asn Trp Tyr Val Asp Gly Val Glu Val His Asn Ala Lys
290 295 300

aca aag ccg cgg gag gag cag tac aac agc acg tac cgt gtg gtc agc 960
Thr Lys Pro Arg Glu Glu Gln Tyr Asn Ser Thr Tyr Arg Val Val Ser
305 310 315 320

gtc ctc acc gtc ctg cac cag gac tgg ctg aat ggc aag gag tac aag 1008
Val Leu Thr Val Leu His Gln Asp Trp Leu Asn Gly Lys Glu Tyr Lys
325 330 335

tgc aag gtc tcc aac aaa gcc ctc cca gcc ccc atc gag aaa acc atc 1056
Cys Lys Val Ser Asn Lys Ala Leu Pro Ala Pro Ile Glu Lys Thr Ile
340 345 350

tcc aaa gcc aaa ggg cag ccc cga gaa cca cag gtg tac acc ctg ccc 1104
Ser Lys Ala Lys Gly Gln Pro Arg Glu Pro Gln Val Tyr Thr Leu Pro
355 360 365

cca tcc cgg gat gag ctg acc aag aac cag gtc agc ctg acc tgc ctg 1152
Pro Ser Arg Asp Glu Leu Thr Lys Asn Gln Val Ser Leu Thr Cys Leu
370 375 380

gtc aaa ggc ttc tat ccc agc gac atc gcc gtg gag tgg gag agc aat 1200
Val Lys Gly Phe Tyr Pro Ser Asp Ile Ala Val Glu Trp Glu Ser Asn

385 390 395 400

ggg cag ccg gag aac aac tac aag acc acg cct ccc gtg ctg gac tcc 1248
Gly Gln Pro Glu Asn Asn Tyr Lys Thr Thr Pro Pro Val Leu Asp Ser
405 410 415

gac ggc tcc ttc ttc ctc tac agc aag ctc acc gtg gac aag agc agg 1296
Asp Gly Ser Phe Phe Leu Tyr Ser Lys Leu Thr Val Asp Lys Ser Arg
420 425 430

tgg cag cag ggg aac gtc ttc tca tgc tcc gtg atg cat gag gct ctg 1344
Trp Gln Gln Gly Asn Val Phe Ser Cys Ser Val Met His Glu Ala Leu
435 440 445

cac aac cac tac acg cag aag agc ctc tcc ctg tct ccg ggt aaa tga 1392
His Asn His Tyr Thr Gln Lys Ser Leu Ser Leu Ser Pro Gly Lys
450 455 460

<210> 10
<211> 463
<212> PRT
<213> Artificial Sequence
<223> Description of Artificial Sequence: Mouse-human chimeric antibody (M3C11 H chain)

<400> 10
Met Asn Phe Gly Leu Thr Leu Ile Phe Leu Val Leu Thr Leu Lys Gly
1 5 10 15

Val Gln Cys Glu Val Gln Leu Val Glu Ser Gly Gly Gly Leu Val Lys
20 25 30

Pro Gly Gly Ser Leu Lys Leu Ser Cys Ala Ala Ser Gly Phe Thr Phe
35 40 45

Ser Arg Tyr Ala Met Ser Trp Val Arg Gln Ile Pro Glu Lys Ile Leu
50 55 60

Glu Trp Val Ala Ala Ile Asp Ser Ser Gly Gly Asp Thr Tyr Tyr Leu
65 70 75 80

Asp Thr Val Lys Asp Arg Phe Thr Ile Ser Arg Asp Asn Ala Asn Asn
85 90 95

Thr Leu His Leu Gln Met Arg Ser Leu Arg Ser Glu Asp Thr Ala Leu
100 105 110

Tyr Tyr Cys Val Arg Gln Gly Gly Ala Tyr Trp Gly Gln Gly Thr Leu
115 120 125

Val Thr Val Ser Ala Ala Ser Thr Lys Gly Pro Ser Val Phe Pro Leu
130 135 140

Ala Pro Ser Ser Lys Ser Thr Ser Gly Gly Thr Ala Ala Leu Gly Cys
145 150 155 160

Leu Val Lys Asp Tyr Phe Pro Glu Pro Val Thr Val Ser Trp Asn Ser

165 170 175

Gly Ala Leu Thr Ser Gly Val His Thr Phe Pro Ala Val Leu Gln Ser
180 185 190

Ser Gly Leu Tyr Ser Leu Ser Ser Val Val Thr Val Pro Ser Ser Ser
195 200 205

Leu Gly Thr Gln Thr Tyr Ile Cys Asn Val Asn His Lys Pro Ser Asn
210 215 220

Thr Lys Val Asp Lys Lys Val Glu Pro Lys Ser Cys Asp Lys Thr His
225 230 235 240

Thr Cys Pro Pro Cys Pro Ala Pro Glu Leu Leu Gly Gly Pro Ser Val
245 250 255

Phe Leu Phe Pro Pro Lys Pro Lys Asp Thr Leu Met Ile Ser Arg Thr
260 265 270

Pro Glu Val Thr Cys Val Val Val Asp Val Ser His Glu Asp Pro Glu
275 280 285

Val Lys Phe Asn Trp Tyr Val Asp Gly Val Glu Val His Asn Ala Lys
290 295 300

Thr Lys Pro Arg Glu Glu Gln Tyr Asn Ser Thr Tyr Arg Val Val Ser
305 310 315 320

Val Leu Thr Val Leu His Gln Asp Trp Leu Asn Gly Lys Glu Tyr Lys
325 330 335

Cys Lys Val Ser Asn Lys Ala Leu Pro Ala Pro Ile Glu Lys Thr Ile
340 345 350

Ser Lys Ala Lys Gly Gln Pro Arg Glu Pro Gln Val Tyr Thr Leu Pro
355 360 365

Pro Ser Arg Asp Glu Leu Thr Lys Asn Gln Val Ser Leu Thr Cys Leu
370 375 380

Val Lys Gly Phe Tyr Pro Ser Asp Ile Ala Val Glu Trp Glu Ser Asn
385 390 395 400

Gly Gln Pro Glu Asn Asn Tyr Lys Thr Thr Pro Pro Val Leu Asp Ser
405 410 415

Asp Gly Ser Phe Phe Leu Tyr Ser Lys Leu Thr Val Asp Lys Ser Arg
420 425 430

Trp Gln Gln Gly Asn Val Phe Ser Cys Ser Val Met His Glu Ala Leu
435 440 445

His Asn His Tyr Thr Gln Lys Ser Leu Ser Leu Ser Pro Gly Lys
450 455 460

<210> 11

<211> 1413

<212> DNA

<213> Artificial Sequence

<220>

<221> CDS

<222> (1).. (1410)

<220>

<223> Description of Artificial Sequence: Mouse-human chimeric antibody (M1E07 H chain)

<400> 11

atg gga tgg aac tgg atc ttt att tta atc ctg tca gta act aca ggt 48
Met Gly Trp Asn Trp Ile Phe Ile Leu Ile Leu Ser Val Thr Thr Gly
1 5 10 15

gtc cac tct gag gtc cag ctg cag cag tct gga cct gag ctg gtg aag 96
Val His Ser Glu Val Gln Leu Gln Gln Ser Gly Pro Glu Leu Val Lys
20 25 30

cct ggg gct tca gtg aag ata tcc tgc aag gct tct ggt tac tca ttc 144
Pro Gly Ala Ser Val Lys Ile Ser Cys Lys Ala Ser Gly Tyr Ser Phe
35 40 45

act ggc tac tac atg cac tgg gtg aag caa agt cct gaa aag agc ctt 192
Thr Gly Tyr Tyr Met His Trp Val Lys Gln Ser Pro Glu Lys Ser Leu
50 55 60

gag tgg att gga gag att aat cct agc act ggt ggt act acc tac aac 240
Glu Trp Ile Gly Glu Ile Asn Pro Ser Thr Gly Gly Thr Thr Tyr Asn
65 70 75 80

cag aag ttc aag gcc aag gcc aca ttg act gta gac aaa tcc tcc agc 288
Gln Lys Phe Lys Ala Lys Ala Thr Leu Thr Val Asp Lys Ser Ser Ser
85 90 95

aca gcc tac atg cag ctc aag agc ctg aca tct gag gac tct gca gtc 336
Thr Ala Tyr Met Gln Leu Lys Ser Leu Thr Ser Glu Asp Ser Ala Val
100 105 110

tat tac tgt gca agg agg ggc gga tta act ggg acg agc ttc ttt gct 384
Tyr Tyr Cys Ala Arg Arg Gly Gly Leu Thr Gly Thr Ser Phe Phe Ala
115 120 125

tac tgg ggc caa ggg act ctg gtc act gtc tct gca gct agc acc aag 432
Tyr Trp Gly Gln Gly Thr Leu Val Thr Val Ser Ala Ala Ser Thr Lys
130 135 140

ggc cca tcg gtc ttc ccc ctg gca ccc tcc tcc aag agc acc tct ggg 480
Gly Pro Ser Val Phe Pro Leu Ala Pro Ser Ser Lys Ser Thr Ser Gly
145 150 155 160

ggc aca gcg gcc ctg ggc tgc ctg gtc aag gac tac ttc ccc gaa ccg 528
Gly Thr Ala Ala Leu Gly Cys Leu Val Lys Asp Tyr Phe Pro Glu Pro
165 170 175

gtg acg gtg tcg tgg aac tca ggc gcc ctg acc agc ggc gtg cac acc 576
Val Thr Val Ser Trp Asn Ser Gly Ala Leu Thr Ser Gly Val His Thr
180 185 190

ttc ccg gct gtc cta cag tcc tca gga ctc tac tcc ctc agc agc gtg 624
Phe Pro Ala Val Leu Gln Ser Ser Gly Leu Tyr Ser Leu Ser Ser Val
195 200 205

gtg acc gtg ccc tcc agc agc ttg ggc acc cag acc tac atc tgc aac 672
Val Thr Val Pro Ser Ser Ser Leu Gly Thr Gln Thr Tyr Ile Cys Asn
210 215 220

gtg aat cac aag ccc agc aac acc aag gtg gac aag aaa gtt gag ccc 720
Val Asn His Lys Pro Ser Asn Thr Lys Val Asp Lys Lys Val Glu Pro
225 230 235 240

aaa tct tgt gac aaa act cac aca tgc cca ccg tgc cca gca cct gaa 768
Lys Ser Cys Asp Lys Thr His Thr Cys Pro Pro Cys Pro Ala Pro Glu
245 250 255

ctc ctg ggg gga ccg tca gtc ttc ctc ttc ccc cca aaa ccc aag gac 816
Leu Leu Gly Gly Pro Ser Val Phe Leu Phe Pro Pro Lys Pro Lys Asp
260 265 270

acc ctc atg atc tcc cgg acc cct gag gtc aca tgc gtg gtg gtg gac 864
Thr Leu Met Ile Ser Arg Thr Pro Glu Val Thr Cys Val Val Val Asp
275 280 285

gtg agc cac gaa gac cct gag gtc aag ttc aac tgg tac gtg gac ggc 912

Val Ser His Glu Asp Pro Glu Val Lys Phe Asn Trp Tyr Val Asp Gly
290 295 300

gtg gag gtg cat aat gcc aag aca aag ccg cgg gag gag cag tac aac 960
Val Glu Val His Asn Ala Lys Thr Lys Pro Arg Glu Glu Gln Tyr Asn
305 310 315 320

agc acg tac cgt gtg gtc agc gtc ctc acc gtc ctg cac cag gac tgg 1008
Ser Thr Tyr Arg Val Val Ser Val Leu Thr Val Leu His Gln Asp Trp
325 330 335

ctg aat ggc aag gag tac aag tgc aag gtc tcc aac aaa gcc ctc cca 1056
Leu Asn Gly Lys Glu Tyr Lys Cys Lys Val Ser Asn Lys Ala Leu Pro
340 345 350

gcc ccc atc gag aaa acc atc tcc aaa gcc aaa ggg cag ccc cga gaa 1104
Ala Pro Ile Glu Lys Thr Ile Ser Lys Ala Lys Gly Gln Pro Arg Glu
355 360 365

cca cag gtg tac acc ctg ccc cca tcc cgg gat gag ctg acc aag aac 1152
Pro Gln Val Tyr Thr Leu Pro Pro Ser Arg Asp Glu Leu Thr Lys Asn
370 375 380

cag gtc agc ctg acc tgc ctg gtc aaa ggc ttc tat ccc agc gac atc 1200
Gln Val Ser Leu Thr Cys Leu Val Lys Gly Phe Tyr Pro Ser Asp Ile
385 390 395 400

gcc gtg gag tgg gag agc aat ggg cag ccg gag aac aac tac aag acc 1248
Ala Val Glu Trp Glu Ser Asn Gly Gln Pro Glu Asn Asn Tyr Lys Thr

405 410 415

acg cct ccc gtg ctg gac tcc gac ggc tcc ttc ttc ctc tac agc aag 1296
Thr Pro Pro Val Leu Asp Ser Asp Gly Ser Phe Phe Leu Tyr Ser Lys
420 425 430

ctc acc gtg gac aag agc agg tgg cag cag ggg aac gtc ttc tca tgc 1344
Leu Thr Val Asp Lys Ser Arg Trp Gln Gln Gly Asn Val Phe Ser Cys
435 440 445

tcc gtg atg cat gag gct ctg cac aac cac tac acg cag aag agc ctc 1392
Ser Val Met His Glu Ala Leu His Asn His Tyr Thr Gln Lys Ser Leu
450 455 460

tcc ctg tct ccg ggt aaa tga 1413
Ser Leu Ser Pro Gly Lys
465 470

<210> 12
<211> 470
<212> PRT
<213> Artificial Sequence
<223> Description of Artificial Sequence: Mouse-human chimeric antibody (M1E07 H chain)

<400> 12
Met Gly Trp Asn Trp Ile Phe Ile Leu Ile Leu Ser Val Thr Thr Gly
1 5 10 15

Val His Ser Glu Val Gln Leu Gln Gln Ser Gly Pro Glu Leu Val Lys
20 25 30

Pro Gly Ala Ser Val Lys Ile Ser Cys Lys Ala Ser Gly Tyr Ser Phe
35 40 45

Thr Gly Tyr Tyr Met His Trp Val Lys Gln Ser Pro Glu Lys Ser Leu
50 55 60

Glu Trp Ile Gly Glu Ile Asn Pro Ser Thr Gly Gly Thr Thr Tyr Asn
65 70 75 80

Gln Lys Phe Lys Ala Lys Ala Thr Leu Thr Val Asp Lys Ser Ser Ser
85 90 95

Thr Ala Tyr Met Gln Leu Lys Ser Leu Thr Ser Glu Asp Ser Ala Val
100 105 110

Tyr Tyr Cys Ala Arg Arg Gly Gly Leu Thr Gly Thr Ser Phe Phe Ala
115 120 125

Tyr Trp Gly Gln Gly Thr Leu Val Thr Val Ser Ala Ala Ser Thr Lys
130 135 140

Gly Pro Ser Val Phe Pro Leu Ala Pro Ser Ser Lys Ser Thr Ser Gly
145 150 155 160

Gly Thr Ala Ala Leu Gly Cys Leu Val Lys Asp Tyr Phe Pro Glu Pro

165 170 175

Val Thr Val Ser Trp Asn Ser Gly Ala Leu Thr Ser Gly Val His Thr
180 185 190

Phe Pro Ala Val Leu Gln Ser Ser Gly Leu Tyr Ser Leu Ser Ser Val
195 200 205

Val Thr Val Pro Ser Ser Ser Leu Gly Thr Gln Thr Tyr Ile Cys Asn
210 215 220

Val Asn His Lys Pro Ser Asn Thr Lys Val Asp Lys Lys Val Glu Pro
225 230 235 240

Lys Ser Cys Asp Lys Thr His Thr Cys Pro Pro Cys Pro Ala Pro Glu
245 250 255

Leu Leu Gly Gly Pro Ser Val Phe Leu Phe Pro Pro Lys Pro Lys Asp
260 265 270

Thr Leu Met Ile Ser Arg Thr Pro Glu Val Thr Cys Val Val Val Asp
275 280 285

Val Ser His Glu Asp Pro Glu Val Lys Phe Asn Trp Tyr Val Asp Gly
290 295 300

Val Glu Val His Asn Ala Lys Thr Lys Pro Arg Glu Glu Gln Tyr Asn
305 310 315 320

Ser Thr Tyr Arg Val Val Ser Val Leu Thr Val Leu His Gln Asp Trp
325 330 335

Leu Asn Gly Lys Glu Tyr Lys Cys Lys Val Ser Asn Lys Ala Leu Pro
340 345 350

Ala Pro Ile Glu Lys Thr Ile Ser Lys Ala Lys Gly Gln Pro Arg Glu
355 360 365

Pro Gln Val Tyr Thr Leu Pro Pro Ser Arg Asp Glu Leu Thr Lys Asn
370 375 380

Gln Val Ser Leu Thr Cys Leu Val Lys Gly Phe Tyr Pro Ser Asp Ile
385 390 395 400

Ala Val Glu Trp Glu Ser Asn Gly Gln Pro Glu Asn Asn Tyr Lys Thr
405 410 415

Thr Pro Pro Val Leu Asp Ser Asp Gly Ser Phe Phe Leu Tyr Ser Lys
420 425 430

Leu Thr Val Asp Lys Ser Arg Trp Gln Gln Gly Asn Val Phe Ser Cys
435 440 445

Ser Val Met His Glu Ala Leu His Asn His Tyr Thr Gln Lys Ser Leu
450 455 460

Ser Leu Ser Pro Gly Lys
465 470

<210> 13

<211> 1416

<212> DNA

<213> Artificial Sequence

<220>

<221> CDS

<222> (1).. (1413)

<220>

<223> Description of Artificial Sequence: Mouse-human chimeric antibody (M19B11 H chain)

<400> 13

```
atg aac ttc ggg ctc acc ttg att ttc ctc gtc ctt act tta aaa ggt    48
Met Asn Phe Gly Leu Thr Leu Ile Phe Leu Val Leu Thr Leu Lys Gly
  1               5                  10                  15

gtc cag tgt gag gtg cag ctg gtg gag tct ggg gga gac tta gtg aag    96
Val Gln Cys Glu Val Gln Leu Val Glu Ser Gly Gly Asp Leu Val Lys
             20                  25                  30

cct gga ggg acc ctg aaa ctc tcc tgt gca gcc tct gga tcc act ttc   144
Pro Gly Gly Thr Leu Lys Leu Ser Cys Ala Ala Ser Gly Ser Thr Phe
         35                  40                  45
```

agt aac tat gcc atg tct tgg gtt cgc cag act cca gag aag agg ctg 192
Ser Asn Tyr Ala Met Ser Trp Val Arg Gln Thr Pro Glu Lys Arg Leu
50 55 60

gag tgg gtc gca gcc att gat agt aat gga ggt acc acc tac tat cca 240
Glu Trp Val Ala Ala Ile Asp Ser Asn Gly Gly Thr Thr Tyr Tyr Pro
65 70 75 80

gac act atg aag gac cga ttc acc att tcc aga gac aat gcc aag aac 288
Asp Thr Met Lys Asp Arg Phe Thr Ile Ser Arg Asp Asn Ala Lys Asn
85 90 95

acc ctg tac ctg caa atg aac agt ctg agg tct gaa gac aca gcc ttt 336
Thr Leu Tyr Leu Gln Met Asn Ser Leu Arg Ser Glu Asp Thr Ala Phe
100 105 110

tat cac tgt aca aga cat aat gga ggg tat gaa aac tac ggc tgg ttt 384
Tyr His Cys Thr Arg His Asn Gly Gly Tyr Glu Asn Tyr Gly Trp Phe
115 120 125

gct tac tgg ggc caa ggg act ctg gtc act gtc tct gca gct agc acc 432
Ala Tyr Trp Gly Gln Gly Thr Leu Val Thr Val Ser Ala Ala Ser Thr
130 135 140

aag ggc cca tcg gtc ttc ccc ctg gca ccc tcc tcc aag agc acc tct 480
Lys Gly Pro Ser Val Phe Pro Leu Ala Pro Ser Ser Lys Ser Thr Ser
145 150 155 160

ggg ggc aca gcg gcc ctg ggc tgc ctg gtc aag gac tac ttc ccc gaa 528

Gly Gly Thr Ala Ala Leu Gly Cys Leu Val Lys Asp Tyr Phe Pro Glu
165 170 175

ccg gtg acg gtg tcg tgg aac tca ggc gcc ctg acc agc ggc gtg cac 576
Pro Val Thr Val Ser Trp Asn Ser Gly Ala Leu Thr Ser Gly Val His
180 185 190

acc ttc ccg gct gtc cta cag tcc tca gga ctc tac tcc ctc agc agc 624
Thr Phe Pro Ala Val Leu Gln Ser Ser Gly Leu Tyr Ser Leu Ser Ser
195 200 205

gtg gtg acc gtg ccc tcc agc agc ttg ggc acc cag acc tac atc tgc 672
Val Val Thr Val Pro Ser Ser Ser Leu Gly Thr Gln Thr Tyr Ile Cys
210 215 220

aac gtg aat cac aag ccc agc aac acc aag gtg gac aag aaa gtt gag 720
Asn Val Asn His Lys Pro Ser Asn Thr Lys Val Asp Lys Lys Val Glu
225 230 235 240

ccc aaa tct tgt gac aaa act cac aca tgc cca ccg tgc cca gca cct 768
Pro Lys Ser Cys Asp Lys Thr His Thr Cys Pro Pro Cys Pro Ala Pro
245 250 255

gaa ctc ctg ggg gga ccg tca gtc ttc ctc ttc ccc cca aaa ccc aag 816
Glu Leu Leu Gly Gly Pro Ser Val Phe Leu Phe Pro Pro Lys Pro Lys
260 265 270

gac acc ctc atg atc tcc cgg acc cct gag gtc aca tgc gtg gtg gtg 864
Asp Thr Leu Met Ile Ser Arg Thr Pro Glu Val Thr Cys Val Val Val

275 280 285

gac gtg agc cac gaa gac cct gag gtc aag ttc aac tgg tac gtg gac 912
Asp Val Ser His Glu Asp Pro Glu Val Lys Phe Asn Trp Tyr Val Asp
290 295 300

ggc gtg gag gtg cat aat gcc aag aca aag ccg cgg gag gag cag tac 960
Gly Val Glu Val His Asn Ala Lys Thr Lys Pro Arg Glu Glu Gln Tyr
305 310 315 320

aac agc acg tac cgt gtg gtc agc gtc ctc acc gtc ctg cac cag gac 1008
Asn Ser Thr Tyr Arg Val Val Ser Val Leu Thr Val Leu His Gln Asp
325 330 335

tgg ctg aat ggc aag gag tac aag tgc aag gtc tcc aac aaa gcc ctc 1056
Trp Leu Asn Gly Lys Glu Tyr Lys Cys Lys Val Ser Asn Lys Ala Leu
340 345 350

cca gcc ccc atc gag aaa acc atc tcc aaa gcc aaa ggg cag ccc cga 1104
Pro Ala Pro Ile Glu Lys Thr Ile Ser Lys Ala Lys Gly Gln Pro Arg
355 360 365

gaa cca cag gtg tac acc ctg ccc cca tcc cgg gat gag ctg acc aag 1152
Glu Pro Gln Val Tyr Thr Leu Pro Pro Ser Arg Asp Glu Leu Thr Lys
370 375 380

aac cag gtc agc ctg acc tgc ctg gtc aaa ggc ttc tat ccc agc gac 1200
Asn Gln Val Ser Leu Thr Cys Leu Val Lys Gly Phe Tyr Pro Ser Asp
385 390 395 400

atc gcc gtg gag tgg gag agc aat ggg cag ccg gag aac aac tac aag 1248
Ile Ala Val Glu Trp Glu Ser Asn Gly Gln Pro Glu Asn Asn Tyr Lys
405 410 415

acc acg cct ccc gtg ctg gac tcc gac ggc tcc ttc ttc ctc tac agc 1296
Thr Thr Pro Pro Val Leu Asp Ser Asp Gly Ser Phe Phe Leu Tyr Ser
420 425 430

aag ctc acc gtg gac aag agc agg tgg cag cag ggg aac gtc ttc tca 1344
Lys Leu Thr Val Asp Lys Ser Arg Trp Gln Gln Gly Asn Val Phe Ser
435 440 445

tgc tcc gtg atg cat gag gct ctg cac aac cac tac acg cag aag agc 1392
Cys Ser Val Met His Glu Ala Leu His Asn His Tyr Thr Gln Lys Ser
450 455 460

ctc tcc ctg tct ccg ggt aaa tga 1416
Leu Ser Leu Ser Pro Gly Lys
465 470

<210> 14
<211> 471
<212> PRT
<213> Artificial Sequence
<223> Description of Artificial Sequence: Mouse-human chimeric antibody (M19B11 H chain)

<400> 14

Met Asn Phe Gly Leu Thr Leu Ile Phe Leu Val Leu Thr Leu Lys Gly
1 5 10 15

Val Gln Cys Glu Val Gln Leu Val Glu Ser Gly Gly Asp Leu Val Lys
20 25 30

Pro Gly Gly Thr Leu Lys Leu Ser Cys Ala Ala Ser Gly Ser Thr Phe
35 40 45

Ser Asn Tyr Ala Met Ser Trp Val Arg Gln Thr Pro Glu Lys Arg Leu
50 55 60

Glu Trp Val Ala Ala Ile Asp Ser Asn Gly Gly Thr Thr Tyr Tyr Pro
65 70 75 80

Asp Thr Met Lys Asp Arg Phe Thr Ile Ser Arg Asp Asn Ala Lys Asn
85 90 95

Thr Leu Tyr Leu Gln Met Asn Ser Leu Arg Ser Glu Asp Thr Ala Phe
100 105 110

Tyr His Cys Thr Arg His Asn Gly Gly Tyr Glu Asn Tyr Gly Trp Phe
115 120 125

Ala Tyr Trp Gly Gln Gly Thr Leu Val Thr Val Ser Ala Ala Ser Thr
130 135 140

Lys Gly Pro Ser Val Phe Pro Leu Ala Pro Ser Ser Lys Ser Thr Ser

145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Lys Val Glu
225 230 235 240

Pro Lys Ser Cys Asp Lys Thr His Thr Cys Pro Pro Cys Pro Ala Pro
245 250 255

Glu Leu Leu Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Glu Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Asp Glu Leu Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 15
<211> 1413
<212> DNA
<213> Artificial Sequence

<220>
<221> CDS
<222> (1)..(1410)

<220>
<223> Description of Artificial Sequence: Mouse-human chimeric antibody (M18D04 H chain)

<400> 15
atg gaa tct aac tgg ata ctt cct ttt att ctg tcg gta gct tca ggg 48
Met Glu Ser Asn Trp Ile Leu Pro Phe Ile Leu Ser Val Ala Ser Gly
1 5 10 15

gtc tac tca gag gtt cag ctc cag cag tct ggg act gtg ctg gca agg 96
Val Tyr Ser Glu Val Gln Leu Gln Gln Ser Gly Thr Val Leu Ala Arg
20 25 30

cct ggg gct tca gtg aag atg tcc tgc aag gct tct ggc tac acc ttt 144

Pro Gly Ala Ser Val Lys Met Ser Cys Lys Ala Ser Gly Tyr Thr Phe
35 40 45

act ggc tac tgg atg cgc tgg gta aaa cag agg cct gga cag ggt ctg 192
Thr Gly Tyr Trp Met Arg Trp Val Lys Gln Arg Pro Gly Gln Gly Leu
50 55 60

gaa tgg att ggc gct att tat cct gga aat agt gat aca aca tac aac 240
Glu Trp Ile Gly Ala Ile Tyr Pro Gly Asn Ser Asp Thr Thr Tyr Asn
65 70 75 80

cag aag ttc aag ggc aag gcc aaa ctg act gca gtc aca tct gtc agc 288
Gln Lys Phe Lys Gly Lys Ala Lys Leu Thr Ala Val Thr Ser Val Ser
85 90 95

act gcc tac atg gaa ctc agc agc ctg aca aat gag gac tct gcg gtc 336
Thr Ala Tyr Met Glu Leu Ser Ser Leu Thr Asn Glu Asp Ser Ala Val
100 105 110

tat tac tgt tca aga tcg ggg gac cta act ggg ggg ttt gct tac tgg 384
Tyr Tyr Cys Ser Arg Ser Gly Asp Leu Thr Gly Gly Phe Ala Tyr Trp
115 120 125

ggc caa ggg act ctg gtc act gtc tct aca gcc aaa gct agc acc aag 432
Gly Gln Gly Thr Leu Val Thr Val Ser Thr Ala Lys Ala Ser Thr Lys
130 135 140

ggc cca tcg gtc ttc ccc ctg gca ccc tcc tcc aag agc acc tct ggg 480
Gly Pro Ser Val Phe Pro Leu Ala Pro Ser Ser Lys Ser Thr Ser Gly

145 150 155 160

ggc aca gcg gcc ctg ggc tgc ctg gtc aag gac tac ttc ccc gaa ccg 528
Gly Thr Ala Ala Leu Gly Cys Leu Val Lys Asp Tyr Phe Pro Glu Pro
165 170 175

gtg acg gtg tcg tgg aac tca ggc gcc ctg acc agc ggc gtg cac acc 576
Val Thr Val Ser Trp Asn Ser Gly Ala Leu Thr Ser Gly Val His Thr
180 185 190

ttc ccg gct gtc cta cag tcc tca gga ctc tac tcc ctc agc agc gtg 624
Phe Pro Ala Val Leu Gln Ser Ser Gly Leu Tyr Ser Leu Ser Ser Val
195 200 205

gtg acc gtg ccc tcc agc agc ttg ggc acc cag acc tac atc tgc aac 672
Val Thr Val Pro Ser Ser Ser Leu Gly Thr Gln Thr Tyr Ile Cys Asn
210 215 220

gtg aat cac aag ccc agc aac acc aag gtg gac aag aaa gtt gag ccc 720
Val Asn His Lys Pro Ser Asn Thr Lys Val Asp Lys Lys Val Glu Pro
225 230 235 240

aaa tct tgt gac aaa act cac aca tgc cca ccg tgc cca gca cct gaa 768
Lys Ser Cys Asp Lys Thr His Thr Cys Pro Pro Cys Pro Ala Pro Glu
245 250 255

ctc ctg ggg gga ccg tca gtc ttc ctc ttc ccc cca aaa ccc aag gac 816
Leu Leu Gly Gly Pro Ser Val Phe Leu Phe Pro Pro Lys Pro Lys Asp
260 265 270

```
acc ctc atg atc tcc cgg acc cct gag gtc aca tgc gtg gtg gtg gac     864
Thr Leu Met Ile Ser Arg Thr Pro Glu Val Thr Cys Val Val Val Asp
        275                 280                 285

gtg agc cac gaa gac cct gag gtc aag ttc aac tgg tac gtg gac ggc     912
Val Ser His Glu Asp Pro Glu Val Lys Phe Asn Trp Tyr Val Asp Gly
    290                 295                 300

gtg gag gtg cat aat gcc aag aca aag ccg cgg gag gag cag tac aac     960
Val Glu Val His Asn Ala Lys Thr Lys Pro Arg Glu Glu Gln Tyr Asn
305                 310                 315                 320

agc acg tac cgt gtg gtc agc gtc ctc acc gtc ctg cac cag gac tgg     1008
Ser Thr Tyr Arg Val Val Ser Val Leu Thr Val Leu His Gln Asp Trp
                325                 330                 335

ctg aat ggc aag gag tac aag tgc aag gtc tcc aac aaa gcc ctc cca     1056
Leu Asn Gly Lys Glu Tyr Lys Cys Lys Val Ser Asn Lys Ala Leu Pro
            340                 345                 350

gcc ccc atc gag aaa acc atc tcc aaa gcc aaa ggg cag ccc cga gaa     1104
Ala Pro Ile Glu Lys Thr Ile Ser Lys Ala Lys Gly Gln Pro Arg Glu
        355                 360                 365

cca cag gtg tac acc ctg ccc cca tcc cgg gat gag ctg acc aag aac     1152
Pro Gln Val Tyr Thr Leu Pro Pro Ser Arg Asp Glu Leu Thr Lys Asn
    370                 375                 380
```

cag gtc agc ctg acc tgc ctg gtc aaa ggc ttc tat ccc agc gac atc 1200
Gln Val Ser Leu Thr Cys Leu Val Lys Gly Phe Tyr Pro Ser Asp Ile
385 390 395 400

gcc gtg gag tgg gag agc aat ggg cag ccg gag aac aac tac aag acc 1248
Ala Val Glu Trp Glu Ser Asn Gly Gln Pro Glu Asn Asn Tyr Lys Thr
405 410 415

acg cct ccc gtg ctg gac tcc gac ggc tcc ttc ttc ctc tac agc aag 1296
Thr Pro Pro Val Leu Asp Ser Asp Gly Ser Phe Phe Leu Tyr Ser Lys
420 425 430

ctc acc gtg gac aag agc agg tgg cag cag ggg aac gtc ttc tca tgc 1344
Leu Thr Val Asp Lys Ser Arg Trp Gln Gln Gly Asn Val Phe Ser Cys
435 440 445

tcc gtg atg cat gag gct ctg cac aac cac tac acg cag aag agc ctc 1392
Ser Val Met His Glu Ala Leu His Asn His Tyr Thr Gln Lys Ser Leu
450 455 460

tcc ctg tct ccg ggt aaa tga 1413
Ser Leu Ser Pro Gly Lys
465 470

<210> 16
<211> 470
<212> PRT
<213> Artificial Sequence

<223> Description of Artificial Sequence: Mouse-human chimeric antibody (M18D04 H chain)

<400> 16

Met Glu Ser Asn Trp Ile Leu Pro Phe Ile Leu Ser Val Ala Ser Gly
1 5 10 15

Val Tyr Ser Glu Val Gln Leu Gln Gln Ser Gly Thr Val Leu Ala Arg
20 25 30

Pro Gly Ala Ser Val Lys Met Ser Cys Lys Ala Ser Gly Tyr Thr Phe
35 40 45

Thr Gly Tyr Trp Met Arg Trp Val Lys Gln Arg Pro Gly Gln Gly Leu
50 55 60

Glu Trp Ile Gly Ala Ile Tyr Pro Gly Asn Ser Asp Thr Thr Tyr Asn
65 70 75 80

Gln Lys Phe Lys Gly Lys Ala Lys Leu Thr Ala Val Thr Ser Val Ser
85 90 95

Thr Ala Tyr Met Glu Leu Ser Ser Leu Thr Asn Glu Asp Ser Ala Val
100 105 110

Tyr Tyr Cys Ser Arg Ser Gly Asp Leu Thr Gly Gly Phe Ala Tyr Trp
115 120 125

Gly Gln Gly Thr Leu Val Thr Val Ser Thr Ala Lys Ala Ser Thr Lys

130 135 140

Gly Pro Ser Val Phe Pro Leu Ala Pro Ser Ser Lys Ser Thr Ser Gly
145 150 155 160

Gly Thr Ala Ala Leu Gly Cys Leu Val Lys Asp Tyr Phe Pro Glu Pro
165 170 175

Val Thr Val Ser Trp Asn Ser Gly Ala Leu Thr Ser Gly Val His Thr
180 185 190

Phe Pro Ala Val Leu Gln Ser Ser Gly Leu Tyr Ser Leu Ser Ser Val
195 200 205

Val Thr Val Pro Ser Ser Ser Leu Gly Thr Gln Thr Tyr Ile Cys Asn
210 215 220

Val Asn His Lys Pro Ser Asn Thr Lys Val Asp Lys Lys Val Glu Pro
225 230 235 240

Lys Ser Cys Asp Lys Thr His Thr Cys Pro Pro Cys Pro Ala Pro Glu
245 250 255

Leu Leu Gly Gly Pro Ser Val Phe Leu Phe Pro Pro Lys Pro Lys Asp
260 265 270

Thr Leu Met Ile Ser Arg Thr Pro Glu Val Thr Cys Val Val Val Asp
275 280 285

Val Ser His Glu Asp Pro Glu Val Lys Phe Asn Trp Tyr Val Asp Gly
290 295 300

Val Glu Val His Asn Ala Lys Thr Lys Pro Arg Glu Glu Gln Tyr Asn
305 310 315 320

Ser Thr Tyr Arg Val Val Ser Val Leu Thr Val Leu His Gln Asp Trp
325 330 335

Leu Asn Gly Lys Glu Tyr Lys Cys Lys Val Ser Asn Lys Ala Leu Pro
340 345 350

Ala Pro Ile Glu Lys Thr Ile Ser Lys Ala Lys Gly Gln Pro Arg Glu
355 360 365

Pro Gln Val Tyr Thr Leu Pro Pro Ser Arg Asp Glu Leu Thr Lys Asn
370 375 380

Gln Val Ser Leu Thr Cys Leu Val Lys Gly Phe Tyr Pro Ser Asp Ile
385 390 395 400

Ala Val Glu Trp Glu Ser Asn Gly Gln Pro Glu Asn Asn Tyr Lys Thr
405 410 415

Thr Pro Pro Val Leu Asp Ser Asp Gly Ser Phe Phe Leu Tyr Ser Lys
420 425 430

Leu Thr Val Asp Lys Ser Arg Trp Gln Gln Gly Asn Val Phe Ser Cys
435 440 445

Ser Val Met His Glu Ala Leu His Asn His Tyr Thr Gln Lys Ser Leu
    450                 455                 460

Ser Leu Ser Pro Gly Lys
465                 470

<210> 17
<211> 717
<212> DNA
<213> Artificial Sequence

<220>
<221> CDS
<222> (1)..(714)

<220>
<223> Description of Artificial Sequence: Mouse-human chimeric antibody (M3C11 L chain)

<400> 17
atg agt cct gcc cag ttc ctg ttt ctg tta gtg ctc tgg att cgg gaa    48
Met Ser Pro Ala Gln Phe Leu Phe Leu Leu Val Leu Trp Ile Arg Glu
  1               5                  10                  15

acc aac ggt gat gtt gtg atg acc cag act cca ctc act ttg tcg gtt    96
Thr Asn Gly Asp Val Val Met Thr Gln Thr Pro Leu Thr Leu Ser Val

20 25 30

acc att gga caa cca gcc tcc atc tct tgc aag tca agt cag agc ctc 144
Thr Ile Gly Gln Pro Ala Ser Ile Ser Cys Lys Ser Ser Gln Ser Leu
35 40 45

tta gat agt gat gga aag aca tat ttg aat tgg ttg tta cag agg cca 192
Leu Asp Ser Asp Gly Lys Thr Tyr Leu Asn Trp Leu Leu Gln Arg Pro
50 55 60

ggc cag tct cca aag cgc cta atc tat ctg gtg tct aaa ttg gac tct 240
Gly Gln Ser Pro Lys Arg Leu Ile Tyr Leu Val Ser Lys Leu Asp Ser
65 70 75 80

gga gcc cct gac agg ttc act ggc agt gga tca ggg aca gat ttc aca 288
Gly Ala Pro Asp Arg Phe Thr Gly Ser Gly Ser Gly Thr Asp Phe Thr
85 90 95

ctg aaa atc agt aga gtg gag gct gag gat ttg gga att tat tat tgc 336
Leu Lys Ile Ser Arg Val Glu Ala Glu Asp Leu Gly Ile Tyr Tyr Cys
100 105 110

tgg caa ggt aca cat ttt ccg ctc acg ttc ggt gct ggg acc aag ctg 384
Trp Gln Gly Thr His Phe Pro Leu Thr Phe Gly Ala Gly Thr Lys Leu
115 120 125

gag ctg aaa cgt acg gtg gct gca cca tct gtc ttc atc ttc ccg cca 432
Glu Leu Lys Arg Thr Val Ala Ala Pro Ser Val Phe Ile Phe Pro Pro
130 135 140

tct gat gag cag ttg aaa tct gga act gcc tct gtt gtg tgc ctg ctg 480
Ser Asp Glu Gln Leu Lys Ser Gly Thr Ala Ser Val Val Cys Leu Leu
145 150 155 160

aat aac ttc tat ccc aga gag gcc aaa gta cag tgg aag gtg gat aac 528
Asn Asn Phe Tyr Pro Arg Glu Ala Lys Val Gln Trp Lys Val Asp Asn
165 170 175

gcc ctc caa tcg ggt aac tcc cag gag agt gtc aca gag cag gac agc 576
Ala Leu Gln Ser Gly Asn Ser Gln Glu Ser Val Thr Glu Gln Asp Ser
180 185 190

aag gac agc acc tac agc ctc agc agc acc ctg acg ctg agc aaa gca 624
Lys Asp Ser Thr Tyr Ser Leu Ser Ser Thr Leu Thr Leu Ser Lys Ala
195 200 205

gac tac gag aaa cac aaa gtc tac gcc tgc gaa gtc acc cat cag ggc 672
Asp Tyr Glu Lys His Lys Val Tyr Ala Cys Glu Val Thr His Gln Gly
210 215 220

ctg agc tcg ccc gtc aca aag agc ttc aac agg gga gag tgt tga 717
Leu Ser Ser Pro Val Thr Lys Ser Phe Asn Arg Gly Glu Cys
225 230 235

<210> 18
<211> 238
<212> PRT

<213> Artificial Sequence

<223> Description of Artificial Sequence: Mouse-human chimeric antibody (M3C11 L chain)

<400> 18

Met Ser Pro Ala Gln Phe Leu Phe Leu Leu Val Leu Trp Ile Arg Glu
1 5 10 15

Thr Asn Gly Asp Val Val Met Thr Gln Thr Pro Leu Thr Leu Ser Val
20 25 30

Thr Ile Gly Gln Pro Ala Ser Ile Ser Cys Lys Ser Ser Gln Ser Leu
35 40 45

Leu Asp Ser Asp Gly Lys Thr Tyr Leu Asn Trp Leu Leu Gln Arg Pro
50 55 60

Gly Gln Ser Pro Lys Arg Leu Ile Tyr Leu Val Ser Lys Leu Asp Ser
65 70 75 80

Gly Ala Pro Asp Arg Phe Thr Gly Ser Gly Ser Gly Thr Asp Phe Thr
85 90 95

Leu Lys Ile Ser Arg Val Glu Ala Glu Asp Leu Gly Ile Tyr Tyr Cys
100 105 110

Trp Gln Gly Thr His Phe Pro Leu Thr Phe Gly Ala Gly Thr Lys Leu
115 120 125

Glu Leu Lys Arg Thr Val Ala Ala Pro Ser Val Phe Ile Phe Pro Pro
130 135 140

Ser Asp Glu Gln Leu Lys Ser Gly Thr Ala Ser Val Val Cys Leu Leu
145 150 155 160

Asn Asn Phe Tyr Pro Arg Glu Ala Lys Val Gln Trp Lys Val Asp Asn
165 170 175

Ala Leu Gln Ser Gly Asn Ser Gln Glu Ser Val Thr Glu Gln Asp Ser
180 185 190

Lys Asp Ser Thr Tyr Ser Leu Ser Ser Thr Leu Thr Leu Ser Lys Ala
195 200 205

Asp Tyr Glu Lys His Lys Val Tyr Ala Cys Glu Val Thr His Gln Gly
210 215 220

Leu Ser Ser Pro Val Thr Lys Ser Phe Asn Arg Gly Glu Cys
225 230 235

<210> 19
<211> 717
<212> DNA
<213> Artificial Sequence

<220>

<221> CDS

<222> (1)..(714)

<220>

<223> Description of Artificial Sequence: Mouse-human chimeric antibody (M1E07 L chain)

<400> 19

```
atg agt cct gtc cag ttc ctg ttt ctg tta atg ctc tgg att cag gaa    48
Met Ser Pro Val Gln Phe Leu Phe Leu Leu Met Leu Trp Ile Gln Glu
 1               5                  10                  15

acc aac ggt gat gtt gtg atg acc cag act cca ctg tct ttg tcg gtt    96
Thr Asn Gly Asp Val Val Met Thr Gln Thr Pro Leu Ser Leu Ser Val
            20                  25                  30

acc att gga caa cca gcc tct atc tct tgc aag tca agt cag agc ctc   144
Thr Ile Gly Gln Pro Ala Ser Ile Ser Cys Lys Ser Ser Gln Ser Leu
        35                  40                  45

tta tat agt aat gga aag aca tat ttg aat tgg tta caa cag agg cct   192
Leu Tyr Ser Asn Gly Lys Thr Tyr Leu Asn Trp Leu Gln Gln Arg Pro
    50                  55                  60

ggc cag gct cca aag cac cta atg tat cag gtg tcc aaa ctg gac cct   240
Gly Gln Ala Pro Lys His Leu Met Tyr Gln Val Ser Lys Leu Asp Pro
65                  70                  75                  80

ggc atc cct gac agg ttc agt ggc agt gga tca gaa aca gat ttt aca   288
```

Gly Ile Pro Asp Arg Phe Ser Gly Ser Gly Ser Glu Thr Asp Phe Thr
85 90 95

ctt aaa atc agc aga gtg gag gct gaa gat ttg gga gtt tat tac tgc 336
Leu Lys Ile Ser Arg Val Glu Ala Glu Asp Leu Gly Val Tyr Tyr Cys
100 105 110

ttg caa agt aca tat tat ccg ctc acg ttc ggt gct ggg acc aag ctg 384
Leu Gln Ser Thr Tyr Tyr Pro Leu Thr Phe Gly Ala Gly Thr Lys Leu
115 120 125

gag ctg aaa cgt acg gtg gct gca cca tct gtc ttc atc ttc ccg cca 432
Glu Leu Lys Arg Thr Val Ala Ala Pro Ser Val Phe Ile Phe Pro Pro
130 135 140

tct gat gag cag ttg aaa tct gga act gcc tct gtt gtg tgc ctg ctg 480
Ser Asp Glu Gln Leu Lys Ser Gly Thr Ala Ser Val Val Cys Leu Leu
145 150 155 160

aat aac ttc tat ccc aga gag gcc aaa gta cag tgg aag gtg gat aac 528
Asn Asn Phe Tyr Pro Arg Glu Ala Lys Val Gln Trp Lys Val Asp Asn
165 170 175

gcc ctc caa tcg ggt aac tcc cag gag agt gtc aca gag cag gac agc 576
Ala Leu Gln Ser Gly Asn Ser Gln Glu Ser Val Thr Glu Gln Asp Ser
180 185 190

aag gac agc acc tac agc ctc agc agc acc ctg acg ctg agc aaa gca 624
Lys Asp Ser Thr Tyr Ser Leu Ser Ser Thr Leu Thr Leu Ser Lys Ala

195 200 205

gac tac gag aaa cac aaa gtc tac gcc tgc gaa gtc acc cat cag ggc 672
Asp Tyr Glu Lys His Lys Val Tyr Ala Cys Glu Val Thr His Gln Gly
210 215 220

ctg agc tcg ccc gtc aca aag agc ttc aac agg gga gag tgt tga 717
Leu Ser Ser Pro Val Thr Lys Ser Phe Asn Arg Gly Glu Cys
225 230 235

<210> 20
<211> 238
<212> PRT
<213> Artificial Sequence
<223> Description of Artificial Sequence: Mouse-human chimeric antibody (M1E07 L chain)

<400> 20
Met Ser Pro Val Gln Phe Leu Phe Leu Leu Met Leu Trp Ile Gln Glu
1 5 10 15

Thr Asn Gly Asp Val Val Met Thr Gln Thr Pro Leu Ser Leu Ser Val
20 25 30

Thr Ile Gly Gln Pro Ala Ser Ile Ser Cys Lys Ser Ser Gln Ser Leu
35 40 45

Leu Tyr Ser Asn Gly Lys Thr Tyr Leu Asn Trp Leu Gln Gln Arg Pro

50 55 60

Gly Gln Ala Pro Lys His Leu Met Tyr Gln Val Ser Lys Leu Asp Pro
65 70 75 80

Gly Ile Pro Asp Arg Phe Ser Gly Ser Gly Ser Glu Thr Asp Phe Thr
85 90 95

Leu Lys Ile Ser Arg Val Glu Ala Glu Asp Leu Gly Val Tyr Tyr Cys
100 105 110

Leu Gln Ser Thr Tyr Tyr Pro Leu Thr Phe Gly Ala Gly Thr Lys Leu
115 120 125

Glu Leu Lys Arg Thr Val Ala Ala Pro Ser Val Phe Ile Phe Pro Pro
130 135 140

Ser Asp Glu Gln Leu Lys Ser Gly Thr Ala Ser Val Val Cys Leu Leu
145 150 155 160

Asn Asn Phe Tyr Pro Arg Glu Ala Lys Val Gln Trp Lys Val Asp Asn
165 170 175

Ala Leu Gln Ser Gly Asn Ser Gln Glu Ser Val Thr Glu Gln Asp Ser
180 185 190

Lys Asp Ser Thr Tyr Ser Leu Ser Ser Thr Leu Thr Leu Ser Lys Ala
195 200 205

Asp Tyr Glu Lys His Lys Val Tyr Ala Cys Glu Val Thr His Gln Gly
210 215 220

Leu Ser Ser Pro Val Thr Lys Ser Phe Asn Arg Gly Glu Cys
225 230 235

<210> 21
<211> 705
<212> DNA
<213> Artificial Sequence

<220>
<221> CDS
<222> (1).. (702)

<220>
<223> Description of Artificial Sequence: Mouse-human chimeric antibody (M19B11 L chain)

<400> 21
atg aga ccc tcc att cag ttc ctg ggg ctc ttg ttg ttc tgg ctt cat 48
Met Arg Pro Ser Ile Gln Phe Leu Gly Leu Leu Leu Phe Trp Leu His
1 5 10 15

ggt gtt cag tgt gac atc cag atg aca cag tct cca tcc tca ctg tct 96
Gly Val Gln Cys Asp Ile Gln Met Thr Gln Ser Pro Ser Ser Leu Ser
20 25 30

gca tct ctg gga ggc aaa gtc acc atc act tgc aag gca agt cag gac 144
Ala Ser Leu Gly Gly Lys Val Thr Ile Thr Cys Lys Ala Ser Gln Asp
35 40 45

att aac aag aat ata gtt tgg tac caa cac aag cct gga aaa ggt cct 192
Ile Asn Lys Asn Ile Val Trp Tyr Gln His Lys Pro Gly Lys Gly Pro
50 55 60

agg ctg ctc ata tgg tac aca tct aca tta cag cca ggc atc cca tca 240
Arg Leu Leu Ile Trp Tyr Thr Ser Thr Leu Gln Pro Gly Ile Pro Ser
65 70 75 80

agg ttc agt gga agt ggg tct ggg aga gat tat tcc ttc agc atc agc 288
Arg Phe Ser Gly Ser Gly Ser Gly Arg Asp Tyr Ser Phe Ser Ile Ser
85 90 95

aac ctg gag cct gaa gat att gca act tat tac tgt cta cag tat gat 336
Asn Leu Glu Pro Glu Asp Ile Ala Thr Tyr Tyr Cys Leu Gln Tyr Asp
100 105 110

aat ctt cca cgg acg ttc ggt gga ggc acc aaa ctg gaa atc aaa cgt 384
Asn Leu Pro Arg Thr Phe Gly Gly Gly Thr Lys Leu Glu Ile Lys Arg
115 120 125

acg gtg gct gca cca tct gtc ttc atc ttc ccg cca tct gat gag cag 432
Thr Val Ala Ala Pro Ser Val Phe Ile Phe Pro Pro Ser Asp Glu Gln
130 135 140

ttg aaa tct gga act gcc tct gtt gtg tgc ctg ctg aat aac ttc tat 480
Leu Lys Ser Gly Thr Ala Ser Val Val Cys Leu Leu Asn Asn Phe Tyr
145 150 155 160

ccc aga gag gcc aaa gta cag tgg aag gtg gat aac gcc ctc caa tcg 528
Pro Arg Glu Ala Lys Val Gln Trp Lys Val Asp Asn Ala Leu Gln Ser
165 170 175

ggt aac tcc cag gag agt gtc aca gag cag gac agc aag gac agc acc 576
Gly Asn Ser Gln Glu Ser Val Thr Glu Gln Asp Ser Lys Asp Ser Thr
180 185 190

tac agc ctc agc agc acc ctg acg ctg agc aaa gca gac tac gag aaa 624
Tyr Ser Leu Ser Ser Thr Leu Thr Leu Ser Lys Ala Asp Tyr Glu Lys
195 200 205

cac aaa gtc tac gcc tgc gaa gtc acc cat cag ggc ctg agc tcg ccc 672
His Lys Val Tyr Ala Cys Glu Val Thr His Gln Gly Leu Ser Ser Pro
210 215 220

gtc aca aag agc ttc aac agg gga gag tgt tga 705
Val Thr Lys Ser Phe Asn Arg Gly Glu Cys
225 230

<210> 22
<211> 234
<212> PRT
<213> Artificial Sequence

<223> Description of Artificial Sequence: Mouse-human chimeric antibody (M19B11 L chain)

<400> 22

Met Arg Pro Ser Ile Gln Phe Leu Gly Leu Leu Leu Phe Trp Leu His
1               5                   10                  15

Gly Val Gln Cys Asp Ile Gln Met Thr Gln Ser Pro Ser Ser Leu Ser
            20                  25                  30

Ala Ser Leu Gly Gly Lys Val Thr Ile Thr Cys Lys Ala Ser Gln Asp
        35                  40                  45

Ile Asn Lys Asn Ile Val Trp Tyr Gln His Lys Pro Gly Lys Gly Pro
    50                  55                  60

Arg Leu Leu Ile Trp Tyr Thr Ser Thr Leu Gln Pro Gly Ile Pro Ser
65                  70                  75                  80

Arg Phe Ser Gly Ser Gly Ser Gly Arg Asp Tyr Ser Phe Ser Ile Ser
                85                  90                  95

Asn Leu Glu Pro Glu Asp Ile Ala Thr Tyr Tyr Cys Leu Gln Tyr Asp
            100                 105                 110

Asn Leu Pro Arg Thr Phe Gly Gly Gly Thr Lys Leu Glu Ile Lys Arg
        115                 120                 125

Thr Val Ala Ala Pro Ser Val Phe Ile Phe Pro Pro Ser Asp Glu Gln

130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys
195 200 205

His Lys Val Tyr Ala Cys Glu Val Thr His Gln Gly Leu Ser Ser Pro
210 215 220

Val Thr Lys Ser Phe Asn Arg Gly Glu Cys
225 230

<210> 23
<211> 720
<212> DNA
<213> Artificial Sequence

<220>
<221> CDS

<222> (1)..(717)

<220>

<223> Description of Artificial Sequence: Mouse-human chimeric antibody (M18D04 L chain)

<400> 23

```
atg agg ttc tct gct cag ctt ctg ggg ctg ctt gtg ctc tgg atc cct     48
Met Arg Phe Ser Ala Gln Leu Leu Gly Leu Leu Val Leu Trp Ile Pro
 1               5                  10                  15

gga tcc act gca gat att gtg atg acg cag gct gca ttc tcc aat cca     96
Gly Ser Thr Ala Asp Ile Val Met Thr Gln Ala Ala Phe Ser Asn Pro
            20                  25                  30

gtc act ctt gga aca tca act tcc atc tcc tgc agg tct agt aag agt    144
Val Thr Leu Gly Thr Ser Thr Ser Ile Ser Cys Arg Ser Ser Lys Ser
        35                  40                  45

ctc cta cat agt aat ggc atc act tat ttg tat tgg tat ctg cag aag    192
Leu Leu His Ser Asn Gly Ile Thr Tyr Leu Tyr Trp Tyr Leu Gln Lys
    50                  55                  60

cca ggc cag tct cct cag ctc ctg att tat cag atg tcc aac ctt gcc    240
Pro Gly Gln Ser Pro Gln Leu Leu Ile Tyr Gln Met Ser Asn Leu Ala
65                  70                  75                  80

tca gga gtc cca gac agg ttc agt agc agt ggg tca gga act gat ttc    288
Ser Gly Val Pro Asp Arg Phe Ser Ser Ser Gly Ser Gly Thr Asp Phe
```

85 90 95

aca ctg aga atc agc aga gtg gag gct gag gat gtg ggt gtt tat tac 336
Thr Leu Arg Ile Ser Arg Val Glu Ala Glu Asp Val Gly Val Tyr Tyr
100 105 110

tgt gct caa aat cta gaa ctt ccg tat acg ttc gga tcg ggg acc aag 384
Cys Ala Gln Asn Leu Glu Leu Pro Tyr Thr Phe Gly Ser Gly Thr Lys
115 120 125

ctg gaa ata aaa cgt acg gtg gct gca cca tct gtc ttc atc ttc ccg 432
Leu Glu Ile Lys Arg Thr Val Ala Ala Pro Ser Val Phe Ile Phe Pro
130 135 140

cca tct gat gag cag ttg aaa tct gga act gcc tct gtt gtg tgc ctg 480
Pro Ser Asp Glu Gln Leu Lys Ser Gly Thr Ala Ser Val Val Cys Leu
145 150 155 160

ctg aat aac ttc tat ccc aga gag gcc aaa gta cag tgg aag gtg gat 528
Leu Asn Asn Phe Tyr Pro Arg Glu Ala Lys Val Gln Trp Lys Val Asp
165 170 175

aac gcc ctc caa tcg ggt aac tcc cag gag agt gtc aca gag cag gac 576
Asn Ala Leu Gln Ser Gly Asn Ser Gln Glu Ser Val Thr Glu Gln Asp
180 185 190

agc aag gac agc acc tac agc ctc agc agc acc ctg acg ctg agc aaa 624
Ser Lys Asp Ser Thr Tyr Ser Leu Ser Ser Thr Leu Thr Leu Ser Lys
195 200 205

gca gac tac gag aaa cac aaa gtc tac gcc tgc gaa gtc acc cat cag 672
Ala Asp Tyr Glu Lys His Lys Val Tyr Ala Cys Glu Val Thr His Gln
210 215 220

ggc ctg agc tcg ccc gtc aca aag agc ttc aac agg gga gag tgt tga 720
Gly Leu Ser Ser Pro Val Thr Lys Ser Phe Asn Arg Gly Glu Cys
225 230 235

<210> 24
<211> 239
<212> PRT
<213> Artificial Sequence
<223> Description of Artificial Sequence: Mouse-human chimeric antibody (M18D04 L chain)

<400> 24
Met Arg Phe Ser Ala Gln Leu Leu Gly Leu Leu Val Leu Trp Ile Pro
1 5 10 15

Gly Ser Thr Ala Asp Ile Val Met Thr Gln Ala Ala Phe Ser Asn Pro
20 25 30

Val Thr Leu Gly Thr Ser Thr Ser Ile Ser Cys Arg Ser Ser Lys Ser
35 40 45

Leu Leu His Ser Asn Gly Ile Thr Tyr Leu Tyr Trp Tyr Leu Gln Lys
50 55 60

Pro Gly Gln Ser Pro Gln Leu Leu Ile Tyr Gln Met Ser Asn Leu Ala
65 70 75 80

Ser Gly Val Pro Asp Arg Phe Ser Ser Ser Gly Ser Gly Thr Asp Phe
85 90 95

Thr Leu Arg Ile Ser Arg Val Glu Ala Glu Asp Val Gly Val Tyr Tyr
100 105 110

Cys Ala Gln Asn Leu Glu Leu Pro Tyr Thr Phe Gly Ser Gly Thr Lys
115 120 125

Leu Glu Ile Lys Arg Thr Val Ala Ala Pro Ser Val Phe Ile Phe Pro
130 135 140

Pro Ser Asp Glu Gln Leu Lys Ser Gly Thr Ala Ser Val Val Cys Leu
145 150 155 160

Leu Asn Asn Phe Tyr Pro Arg Glu Ala Lys Val Gln Trp Lys Val Asp
165 170 175

Asn Ala Leu Gln Ser Gly Asn Ser Gln Glu Ser Val Thr Glu Gln Asp
180 185 190

Ser Lys Asp Ser Thr Tyr Ser Leu Ser Ser Thr Leu Thr Leu Ser Lys
195 200 205

Ala Asp Tyr Glu Lys His Lys Val Tyr Ala Cys Glu Val Thr His Gln

210 215 220

Gly Leu Ser Ser Pro Val Thr Lys Ser Phe Asn Arg Gly Glu Cys

225 230 235

## Description

### Technical Field

The present invention relates to an antibody against an N-terminal peptide or C-terminal peptide of GPC3. More specifically, the invention relates to an antibody against a GPC3 N-terminal peptide of about 40 kDa as found in the soluble form of the GPC3 core protein. Additionally, the invention also relates to an antibody against a GPC3 C-terminal peptide of about 30 kDa as found in the soluble form of the GPC3 core protein.

### Background Art

The presence of the glypican family is reported as a new family of heparan sulfate proteoglycan existing on cell surface. Up to now, it is reported that five types of glypican (glypican 1, glypican 2, glypican 3, glypican 4 and glypican 5) exist. The members of the family have a core protein of a uniform size (about 60 kDa) and have unique cysteine residues well conserved in common, and are bound to cell membrane via glycosylphosphatidylinositol (GPI) anchor.

Glypican 3 (GPC3) is known to be deeply involved in cell division during development and the control of the pattern thereof. Additionally, it is known that the GPC3 gene is highly expressed in hepatoma cell and that the GPC3 gene is possibly used as a marker of hepatocellular carcinoma.

The present inventors previously found that an anti-GPC3 antibody had an ADCC activity and a CDC activity and was useful as the therapeutic treatment of hepatoma and filed a patent application (Japanese Patent Application 2001-189443).

However, GPC3 is a membrane-bound protein and it has not been reported that a GPC3 protein of secreted form existed. Thus, no examination has been made about the use of the GPC3 protein itself as a tumor marker in blood.

### Disclosure of the Invention

The present inventors found a fact that glypican 3 (GPC3) is cleaved at an amino acid residue 358 thereof or at an amino acid residue 374 thereof or a region in the vicinity of the residues. On an assumption that the soluble form of GPC3 would be secreted in the blood of hepatoma patients, the inventors established a GPC3 sandwich ELISA system to show the existence of the secreted form of GPC3 in the culture supernatant of human hepatoma cell HepG2 highly expressing GPC3. Further, the inventors successfully assayed the secreted form of GPC3 not only in the plasma of a mouse transplanted with HepG2 but also in the serumof a human hepatoma patient. Because the expression of the GPC3 gene is observed in hepatoma at an earlier stage compared with the time involving the occurrence of AFP as a hepatoma marker, the inventors considered that the detection of GPC3 would be useful for cancer diagnosis. Additionally because it appears to be hard to detect the secreted form of GPC3 with an anti-GPC3 antibody recognizing a C-terminal peptide fragment, the secreted form of GPC3 was assumed to be dominantly present as an N-terminal peptide fragment. Thus, the inventors considered that an anti-GPC3 antibody recognizing the N terminus was preferably used for detecting the secreted form of GPC3. Accordingly, the inventors made an attempt to develop an antibody recognizing the N-terminal peptide of GPC3, and thus have achieved the invention. Further, the inventors found that an antibody against the C terminus of GPC3 had a high cytotoxic activity and considered that the use of the anti-GPC3 antibody recognizing the C terminus would be preferable for disrupting cancer cell, i.e. for therapeutically treating cancer. Then, the inventors made an attempt of developing an antibody recognizing the C-terminal peptide of GPC3, and thus have achieved the invention.

Since it is observed that GPC3 is expressed in cancer cell lines other than hepatoma cell lines, such as lung cancer, colon cancer, breast cancer, prostate cancer, pancreatic cancer, and lymphoma, GPC3 may possibly be applied to the diagnosis of cancers other than hepatoma.

Specifically, the invention relates to an antibody against an N-terminal peptide of GPC3.

Additionally, the invention relates to the antibody, where the N-terminal peptide of GPC3 is a secreted form of a peptide found in blood.

Further, the invention relates to the antibody, where the N-terminal peptide of GPC3 is a peptide comprising amino acid residues 1-374 of GPC3 or a peptide comprising amino acid residues 1-358 of GPC3.

Still further, the invention relates to the antibody, which is a monoclonal antibody.

Additionally, the invention relates to the antibody, which is immobilized to an insoluble support.

Still additionally, the invention relates to the antibody, which is labeled with a labeling material.

Still more additionally, the invention relates to an antibody against a C-terminal peptide of GPC3.

Still further, the invention relates to the antibody, where the C-terminal peptide of GPC3 is a peptide comprising amino acid residues 359-580 of GPC3 or a peptide comprising amino acid residues 375-580 of GPC3.

Still further, the invention relates to the antibody, which is a monoclonal antibody.

Additionally, the invention relates to the antibody, which is a chimera antibody.

Additionally, the invention relates to the antibody, which is a cytotoxic antibody.

Still additionally, the invention relates to a cell-disrupting agent comprising the antibody.

Additionally, the invention relates to the cell disrupting agent, where the cell is a cancer cell.

Further, the invention relates to an anti-cancer agent comprising the antibody.

Additionally, the invention relates to a method for inducing cytotoxicity comprising contacting a cell with the antibody.

Still more additionally, the invention relates to the method, where the cell is a cancer cell.

The invention is now described in detail hereinbelow.

The invention provides an antibody against the secreted form of glypican 3 (GPC3), which is capable of detecting the secreted formof GPC3 in a test sample. By detecting the secreted form of GPC3 in vitro in a test sample, it can be diagnosed whether or not the test subject is afflicted with cancer, particularly hepatoma.

Detection includes quantitative or non-quantitative detection, and includes for example a simple assay for the existence of GPC3 protein, an assay for the existence of GPC3 protein at a given amount or more, and a comparative assay for the amount of GPC3 protein with the amount in other samples (for example, control sample) as a non-quantitative assay; and an assay for the concentration of the GPC3 protein and an assay for the amount of the GPC3 protein as a quantitative assay.

The test sample includes, but is not limited to, any samples possibly containing the GPC3 protein. A sample collected from biological bodies of mammals is preferable. Further, samples collected from humans are more preferable. Specific examples of such test sample include blood, interstitial fluid, plasma, extravascularfluid,cerebrospinal fluid, synovial fluid, pleural fluid, serum, lymphoid fluid, saliva, and urine. Preferably, the test sample is blood, serum or plasma. Additionally, samples obtained from test samples, such as a culture medium of cells collected from biological bodies are also included in the test sample in accordance with the invention.

The cancer to be diagnosed using the antibody against the N-terminal peptide of GPC3 in accordance with the invention includes, but is not limited to, hepatoma, pancreatic cancer, lung cancer, colon cancer, breast cancer, prostate cancer, leukemia, and lymphoma. Preferably, the cancer is hepatoma.

Because the antibody against the C-terminal peptide of GPC3 in accordance with the invention has a high cytotoxic activity, the antibody can be used for disrupting cancer cells, i.e. for therapeutically treating cancer. Cancer possibly treated clinically using the antibody includes, but is not limited to, hepatoma, pancreatic cancer, lung cancer, colon cancer, breast cancer, prostate cancer,leukemia,andlymphoma. Preferably, the cancer is hepatoma.

1. Preparation of the anti-GPC3 antibody against the N-terminal peptide or the anti-GPC3 antibody against the C-terminal peptide

The amino acid sequence and nucleotide sequence of GPC3 are described in Lage, H. et al., Gene 188 (1997), 151-156 or GenBank: Z37987.

The anti-GPC3 antibody against the N-terminal peptide or the anti-GPC3 antibody against the C-terminal peptide used in the invention should be capable of specifically binding to the N-terminal peptide of the GPC3 protein or the C-terminal peptide of the GPC3 protein, respectively. The origin or type thereof (monoclonal, polyclonal) or the shape thereof is not specifically limited. Specifically, known antibodies such as mouse antibody, rat antibody, human antibody, chimera antibody and humanized antibody can be used.

When GPC3 is cleaved at a cleavage site, the GPC3 is cut into a peptide of about 40 kDa and a peptide of about 30 kDa, which are on the N-terminal side and the C-terminal side, respectively. The cleavage site of GPC3 is the amino acid reside 358, the amino acid residue 374 or a region in the vicinity thereof. The main cleavage site is believed to be the amino acid residue 358.

The N-terminal peptide of GPC3 is an N-terminal peptide of GPC3 and of about 40 kDa, which is found in the soluble form of the GPC3 core protein. The N-terminal peptide is preferably a peptide of an amino acid sequence comprising from Met 1 to Lys 374, or a peptide of an amino acid sequence comprising from Met 1 to Arg 358. More preferably, the N-terminal peptide is a peptide of an amino acid sequence comprising from Met 1 to Arg 358, because the main cleavage site is predicted to be at the amino acid residue 358. In accordance with the invention, fragments of the N-terminal peptide may also be employed. In this specification, the N-terminal peptide is also referred to as N-terminal fragment or N-terminal peptide fragment.

In other words, the antibody against the N-terminal peptide of GPC3 in accordance with the invention is an antibody recognizing an epitope existing on the N-terminal peptide of the GPC3 protein. The site of the epitope recognized is not specifically limited.

The C-terminal peptide of GPC3 is a C-terminal peptide of GPC3 and of about 30 kDa found in the soluble form of the GPC3 core protein. Based on the cleavage site mentioned above, the C-terminal peptide is preferably a peptide of an amino acid sequence of from Ser 359 to His 580 or a peptide of an amino acid sequence of from Val 375 to His 580. More preferably, the C-terminal peptide is a peptide of an amino acid sequence comprising from Ser 359 to His 580, because the main cleavage site is presumed to be at the site of the amino acid residue 358. In accordance with the invention, fragments of such C-terminal peptide may also be employed. In this specification, the C-terminal peptide is also referred to C-terminal fragment or C-terminal peptide fragment.

In other words, the antibody against the C-terminal peptide of GPC3 in accordance with the invention is an antibody recognizing an epitope existing on the C-terminal peptide of the GPC3 protein, and the site of the epitope recognized is not limited.

The antibodymay be a polyclonal antibody but is preferably a monoclonal antibody.

The anti-GPC3 N-terminal peptide antibody or the anti-GPC3 C-terminal peptide antibody for use in accordance with the invention can be obtained as a polyclonal antibody or a monoclonal antibody, using known techniques. The anti-GPC3 antibody for use in accordance with the invention is preferably a monoclonal antibody derived from mammals. The monoclonal antibody derived from mammals includes those produced by hybridoma, and those generated in hosts transformed with expression vectors carrying the antibody gene by genetic engineering technology.

Hybridoma producing a monoclonal antibody is prepared essentially using known techniques as follows. An animal is immunized by a conventional immunization method using GPC3 as a sensitizing antigen to obtain an immune cell, which is then fused to a known parent cell by a conventional cell fusion method. Fused cells are screened for monoclonal antibody-generating cells by a conventional screening method.

Specifically, a monoclonal antibody is prepared as follows.

First, GPC3 for use as a sensitizing antigen for obtaining antibody is prepared by expressing the GPC3 (MXR7) gene/amino acid sequence disclosed in Lage, H. et al., Gene 188 (1997), 151-156. Particularly, the gene sequence encoding GPC3 is inserted in a knownexpression vector to transform an appropriate host cell, then the intended human GPC3 protein is purified from the host cell or a culture supernatant thereof.

Additionally, naturally occurring GPC3 may also be purified and used.

Then, the purified GPC3 protein is used as a sensitizing antigen. The whole GPC3 protein may be used as a sensitizing antigen. Because an antibody against the N-terminal peptide of the GPC3 protein and an antibody against the C-terminal peptide thereof are also induced in this case, the antibody against the N-terminal peptide of the GPC3 protein and the antibody against the C-terminal peptide thereof may be separately selected. Alternatively, a partial N-terminal peptide of GPC3 or a partial C-terminal peptide thereof may also be used as a sensitizing antigen. Inthatcase, such partial peptide may be obtained by chemical synthesis on the basis of the amino acid sequence of human GPC3 or by inserting a part of the GPC3 gene into an expression vector or by degrading naturally occurring GPC3 with proteases. The part of GPC3 for use as a partial peptide is the N-terminal GPC3 peptide. A smaller peptide fragment containing the epitope in the part may also be used. Further, a C-terminal peptide of GPC3 may be used as a partial peptide, and a smaller peptide fragment containing the epitope in the part may also be used.

Mammals for immunization with a sensitizing antigen are preferably selected, with taking account of the compatibility with parent cells for use in cell fusion. The mammals used for immunization preferably include, but are not limited to, rodents such as mouse, rat, hamster or rabbit or monkey.

For immunization of animals with a sensitizing antigen, known methods may be employed. Generally, for example, a sensitizing antigen is injected intraperitoneally or subcutaneously in mammals. Specifically, a sensitizing antigen is diluted with or suspended in PBS (phosphate-buffered saline) or physiological saline or the like, to an appropriate volume, and mixed with an appropriate volume of conventional adjuvants, such as Freund's complete adjuvant. After emulsification, the emulsified mixture is administered to mammals several times every 4 to 21 days. Additionally, an appropriate carrier may be used during the immunization with a sensitizing antigen. In case that a partial peptide of a very small molecular weight is to be used as a sensitizing antigen, the partial peptide may preferably be bound to carrier proteins, such as albumin and keyhole limpet hemocyanin upon immunization.

After mammals are immunized as above and the increase in the level of a desired antigen in serum is observed, immune cells are collected from the mammals, which are then subjected to cell fusion. Preferably, the immune cell is splenocyte.

As another parent cell to be fused to the immune cell, mammalian myeloma cell may be used. As the myeloma cell, known various cell lines are preferably used, including for example P3 (P3x63Ag8. 653) (J. Immunol. (1979) 123, 1548-1550), P3x63Ag8U. 1 (Current Topics in Microbiology and Immunology (1978) 81, 1-7), NS-1 (KohlerG. andMilstein, C. Eur. J. Immunol. (1976) 6, 511-519), MPC-11 (Margulies, D. H. etal., Cell (1976) 8, 405-415), SP2/0 (Shulman, M. et al., Nature (1978) 276, 269-270), F0 (de St. Groth, S. F. et al., J. Immunol. Methods (1980) 35, 1-21), S194 (Trowbridge, I. S. J. Exp. Med. (1978) 148, 313-323), and R210 (Galfre, G. et al., Nature (1979) 277, 131-133).

The cell fusion of the immune cell to the myeloma cell is essentially done by known methods, for example the method of Kohler & Milstein et al. ( Kohler G. and Milstein C., Methods Enzymol. (1981) 73, 3-46).

More specifically, the cell fusion is carried out in conventional nutritious culture media in the presence of a cell fusion stimulator. Cell fusion stimulator includes, for example, polyethylene glycol (PEG) and Sendai virus (HVJ) . If desired, auxiliary agents such as dimethylsulfoxide can be added and used so as to enhance the fusion efficiency.

The ratio of an immune cell and a myeloma cell to be used can appropriately be determined. For example,'an immune cell at a ratio of 1- to 10-fold a myeloma cell is preferable. Culture medium for use in the cell fusion includes, for example, RPMI1640 and MEM, and other conventional culture media suitable for the growth of myeloma cell lines. Further, auxiliary serum agents such as fetal calf serum (FCS) may be used in combination.

The cell fusion can be done by thoroughly mixing predetermined amounts of immune cells and myeloma cells in the culture medium, adding the resulting mixture to a PEG solution (for example, mean molecular weight of about 1,000 to 6,000) preliminarily heated to about 37 °C, generally to a concentration of 30 to 60 w/v %, and subsequently mixing the mixture to allow the intended fusion cell (hybridoma) to be formed. Subsequently, a cell fusion agent and the like unpreferable for the growth of hybridoma are removed by adding appropriate culture medium sequentially and centrifuging the mixture to discard the supernatant, and repeating the procedures described above.

The hybridoma thus obtained is selected by culturing in a conventional selective culture medium, such as HAT medium (containing hypoxanthine, aminopterin and thymidine). The culturing in the HAT medium is continued for a sufficient period of time (typically several days to several weeks) for killing cells (non-fused cells) other than the intended hybridoma cell. Then, a conventional limited dilution method is carried out for screening and single cloning of a hybridoma producing the intended antibody.

The screening and the single cloning of the hybridoma may be done by a screening method on the basis of known antigen-antibody reactions. The antigen is bound to carriers such as beads made of polystyrene and the like, or commercially available 96-well microtiter plates, and reacted with a culture supernatant of the hybridoma. After rinsing the carriers, an enzyme-labeled secondary antibody is added to the plate to determine whether an intended antibody reacting with the sensitizing antigen is contained in the culture supernatant. The hybridoma producing the intended antibody can be cloned by limited dilution method. The N-terminal peptide of GPC3 or a fragment thereof or the C-terminal peptide of GPC3 or a fragment thereof may be used as the antigen for screening.

In addition to obtaining hybridoma by immunizing an animal except humans with an antigen, a human antibody may be prepared by another method. Human lymphocyte is sensitized with GPC3 in vitro and is then fused to myeloma cell with a permanent division potency derived from humans, to obtain a desired human antibody with a binding activity to the N-terminal peptide of GPC3 or the C-terminal peptide of GPC3 (see JP-B-1-59878). Further, a human antibody against the N-terminal peptide of GPC3 or the C-terminal peptide of GPC3 may be obtained by administering GPC3 as an antigen to a transgenic animal bearing all the repertories of the genes of human antibodies to obtain a cell producing an anti-GPC3 antibody against the N-terminal peptide or a cell producing an anti-GPC3 antibody against the C-terminal peptide, and then immortalizing the cell (see International Publications WO 94/25585, WO 93/12227, WO 92/03918, and WO 94/02602).

The hybridoma producing the monoclonal antibody thus prepared can be subcultured in a conventional culture medium and can be stored in liquid nitrogen for a long period of time.

One method for obtaining the monoclonal antibody from the hybridoma involves culturing the hybridoma by a conventional method and obtaining the monoclonal antibody from a culture supernatant thereof. Another method involves administering the hybridoma to an animal compatible to the hybridoma for proliferation and obtaining the monoclonal antibody in the form of ascites. The former method is suitable for obtaining the antibody at high purity, while the latter method is suitable for large-scale production of the antibody.

In accordance with the invention, a monoclonal antibody includes a recombinant antibody produced by gene recombinant technology. A recombinant antibody can be generated by cloning the gene of the antibody from the hybridoma, integrating the gene into an appropriate vector, introducing the gene into a host, and allowing the recombinant antibody to be produced by the host (see for example Vandamme, A. M. et al. , Eur. J. Biochem. (1990) 192, 767-775, 1990). Specifically, mRNA encoding the variable (V) region of the anti-GPC3 N-terminal peptide or the anti-GPC3 C-terminal peptide is isolated from the hybridoma generating the anti-GPC3 N-terminal peptide antibody or the hybridoma generating the anti-GPC3 C-terminal peptide antibody, respectively. mRNA isolation can be done by known methods. For example, total RNA is prepared by guanidine ultra-centrifugation method (Chirgwin, J. M. et al., Biochemistry (1979) 18, 5294-5299) or AGPC method (Chomczynski, P. et al., Anal. Biochem. (1987) 162, 156-159), from which the intended mRNA is prepared using the mRNA purification kit (manufactured by Pharmacia). Alternatively, mRNA can directly be prepared using QuickPrep mRNA purification kit (manufactured by Pharmacia).

cDNA of the V region of the antibody is synthesized from the resulting mRNA, using reverse transcriptase. cDNA can be synthesized, using AMV Reverse Transcriptase First-strand cDNA Synthesis Kit (manufactured by Seikagaku Corporation). cDNA can also be synthesized and amplified using 5'-AmpliFinder Race Kit (manufactured by Clontech) and 5'-RACE method using PCR (Frohman, M.A. et al., Proc. Natl. Acad. Sci. USA (1988) 85, 8998-9002; Belyavsky, A. et al., Nucleic Acids Res. (1989) 17, 2919-2932).

The intended DNA fragment is purified from the resulting PCR product and linked to vector DNA. A recombinant vector is prepared from the vector DNA and introduced in Escherichia coli and the like to select a colony for preparation of a desired recombinant vector. Subsequently, the nucleotide sequence of the intended DNA can be confirmed by known methods, for example dideoxynucleotide chain termination method.

After DNA encoding the V region of the intended anti-GPC3 N-terminal peptide antibody or the intended anti-GPC3 C-terminal peptide antibody is obtained, the DNA is inserted into an expression vector containing DNA encoding the desired constant region (C region) of the antibody.

So as to produce the anti-GPC3 N-terminal peptide antibody or the anti-GPC3 C-terminal peptide antibody for use in accordance with the invention, the gene of the antibody is introduced into an expression vector such that the gene is expressed underthecontrolofanexpression-regulating region, for example enhancer and promoter. Then, a host cell is transformed with the expression vector, to express the antibody.

The gene of the antibody may be expressed by separately inserting DNA encoding the heavy chain (H chain) of the antibody and DNA encoding the light chain (L chain) thereof in expression vectors to simultaneously transform a host cell, or by inserting DNAs encoding the H chain and the L chain in a single expression vector to transform a host cell (see WO 94/11523).

Additionally, not only such host cells but also transgenic animal can be used for generating a recombinant antibody. For example, the gene of the antibody is inserted intermediately into a gene encoding a protein (e.g., goat β casein) generated inherently in milk to prepare a fusion gene. The DNA fragment comprising the fusion gene with the gene of the antibody as inserted therein is injected in a goat embryo, which is introduced in a female goat. The desired antibody is obtained from the milk produced by a transgenic goat born from the goat having received the embryo or a progeny thereof. So as to increase the amount of milk containing the desired antibody as produced by the transgenic goat, hormone may appropriately be administered to the transgenic goat (Ebert, K. M. et al., Bio/Technology (1994) 12, 699-702)

In accordance with the invention, artificially modified recombinant antibodies, for example a chimera antibody (e.g., humanized antibody) may also be used. These modified antibodies can be produced, using existing methods. In case that the antibody of the invention is to be used as an antibody for therapeutic treatment, the genetic recombinant type antibody is preferably used.

Chimera antibody can be obtained by linking the DNA encoding the V region of the antibody as obtained in the manner described above to DNA encoding the C region of a human antibody, inserting the resulting DNA in an expression vector, and introducing the vector in a host for production of the antibody. Using this existing method, a chimera antibody useful in accordance with the invention can be obtained.

Humanized antibody is also referred to as reshaped human antibody and is prepared by transplanting the complementarity determining region (CDR) of an antibody of mammals except humans, for example mouse, into the complementarity determining region of a human antibody. General genetic recombination techniques thereof are also known in the art (see European Patent Application EP 125023; WO 96/02576).

Specifically, a DNA sequence designed such that the CDR of mouse antibody can be linked to the framework region (FR) of human antibody is synthetically prepared by PCR, using several oligonucleotides prepared in such a manner that the oligonucleotides might have parts overlapped with the terminal regions of both CDR and FR (see the method described in WO 98/13388).

The FR region of human antibody to be liked to CDR is selected such that the CDR can form a good antigen binding site. If necessary, the amino acids in the FR in the V region of the antibody may be substituted, so that the CDR of the reshaped human antibody may form an appropriate antigen binding site (Sato, K. et al., Cancer Res. (1993) 53, 851-856).

As the C regions of chimera antibody and humanized antibody, those of human antibody are used; for example, Cγ1, Cγ2, Cγ3, and Cγ4 can be used for the H chain, while Cκ and Cλ can be used for the L chain. So as to improve the stability of the antibody or the production thereof, the C region of human antibody may be modified.

Preferably, the chimera antibody contains a sequence of an antibody derived from mammals except humans in the V region, and contains a sequence derived from a human antibody in the C region.

Humanized antibody comprises the CDR of an antibody derived from mammals except humans, and the FR and C regions derived from a human antibody. Because the antigenicity of chimera antibody such as humanized antibody is reduced in humans, chimera antibody is useful as an active component of a therapeutic agent of the invention.

The antibody for use in accordance with the invention is not only the whole antibody molecule but also a fragment of the antibody or a modified product thereof, including divalent antibody and monovalent antibody, as long as such fragment or such modified product can bind to the GPC3 N-terminal peptide or the GPC3 C-terminal peptide. For example, the antibody fragment includes Fab, F(ab')2, Fv, Fab/C having one Fab and complete FC, or single chain Fv (scFv) where Fv of the H chain and the L chain are linked via an appropriate linker. Specifically, the antibody is treated with enzymes, for example papain and pepsin, to generate antibody fragments. Otherwise, genes encoding these antibody fragments are constructed, introduced in an expression vector and expressed in an appropriate host cell (see for example, Co, M. S. et al., J. Immunol. (1994) 152, 2968-2976; Better, M. & Horwitz, A. H. Methods in Enzymology (1989) 178, 476-496, Academic Press, Inc.; Plueckthun, A. & Skerra, A. Methods in Enzymology (1989) 178, 476-496, Academic Press, Inc.; Lamoyi, E., Methods in Enzymology (1989) 121, 652-663; Rousseaux, J. et al., Methods in Enzymology (1989) 121, 663-669; Bird, R. E. et al., TIBTECH (1991) 9, 132-137).

ScFv can be obtained by linking the V region of the H chain and the V region of the L chain of an antibody. In this scFv, the V region of the H chain and the V region of the L chain are linked together via a linker, preferably a peptide linker (Huston, J. S. et al., Proc. Natl. Acad. Sci. U.S.A. (1988) 85, 5879-5883). The V region of the H chain and the V region of the L chain in scFv may be derived from any antibodies described herein. Any appropriate single-stranded peptide comprising 12 to 19 amino acid residues may be used as the peptide linker for linking the V regions.

DNA encoding scFv is obtained by first amplifying DNA encoding the H chain or the V region of the H chain and the DNA encoding the L chain or the V region of the L chain by using as a template a portion of DNA encoding all the sequences thereof or a desired amino acid sequence therein and a pair of primers defining both the ends, and then amplifying the DNA with DNA encoding the peptide linker and a pair of primers defined in such a manner that both the ends of the peptide linker may be linked respectively to the H chain and the L chain.

Once the DNA encoding scFv is prepared, an expression vector carrying the DNA and a host transformed with the expression vector can be obtained by conventional methods. scFv can be obtained using the host by conventional methods.

The antibody fragments can be generated by obtaining and expressing the gene in the same manner as described above and allowing a host to produce the fragments. The "antibody" in accordance with the invention includes such antibody fragments.

There may also be used a modified product of the antibody, for example, anti-glypican antibodies conjugated with various molecules such as labeling substances, toxin, and radioactive materials. The "antibody" in accordance with the invention includes these modified antibodies. Such modified antibodies can be obtained by chemical modification of an antibody. Methods for modifying antibodies have already been established in the art.

Further, the antibody for use in accordance with the invention may be a bispecific antibody. The bispecific antibody may include those having antigen binding sites recognizing different epitopes on the N-terminal peptide of GPC3 or the C-terminal peptide of GPC3. Alternatively, one of the antigen binding sites recognizes the N-terminal peptide of GPC3 or the C-terminal peptide of GPC3, while the other antigen binding site may recognize a labeling substance and the like. Such bispecific antibody can be prepared or obtained by linking HL pairs of two types of antibodies or by fusing hybridomas generating different monoclonal antibodies together to prepare a fusion cell capable of producing a bispecific antibody. Further, such bispecific antibody can be prepared by genetic engineering technique.

In accordance with the invention, an antibody with a modified sugar chainmay also be used for the purpose of enhancing cytotoxic activity. Modification technique of the sugar chain of antibody is known in the art (for example, WO 00/61739, WO 02/31140, etc.).

The antibody gene constructed in the manner described above can be expressed and obtained by known methods. In case of a mammalian cell, a conventional useful promoter, the antibody gene to be expressed and poly (A) signal downstream the 3' side thereof are functionally linked for the expression. For example, the promoter /enhancer includes human cytomegalovirus immediate early promoter/enhancer.

Additionally, the promoter/enhancer for use in the expression of the antibody for use in accordance with the invention includes, for example, virus promoters including retrovirus, polyoma virus, adenovirus and simian virus 40 (SV40) /enhancer or promoters derived from mammalian cells such as human elongation factor Ia (HEFIa)/enhancer.

In case of using SV40 promoter/enhancer, gene expression can readily be done by the method of Mulligan et al. (Nature (1979) 277,108). In case of using the HEFI a promoter/enhancer, gene expression can readily be done by the method of Mizushima et al. (Nucleic Acids Res. (1990) 18, 5322).

In case of Escherichia coli, a useful conventional promoter, a signal sequence for antibody secretion and an antibody gene to be expressed are functionally linked for expressing the gene. The promoter includes for example lacz promoter and araB promoter. In case that lacz promoter is to be used, the gene can be expressed by the method of Ward et al. (Nature (1098), 341, 544-546; FASEBJ. (1992) 6, 2422-2427). In case that araB promoter is to be used, the gene can be expressed by the method of Better et al. (Science (1988) 240, 1041-1043).

As the signal sequence for antibody secretion, pelB signal sequence (Lei, S. P. et al. J. Bacteriol. (1987) 169, 4379) may be used when the antibody is generated in the periplasm of Escherichia coli. After the antibody generated in the periplasm is separated, the structure of the antibody is appropriately refolded for use.

As the replication origin, those from SV40, polyoma virus, adenovirus and bovine papilloma virus (BPV) may be used. For amplification of the copy number of the gene in a host cell system, the expression vector may carry a selective marker, for example, aminoglycoside transferase (APH) gene, thymidine kinase (TK) gene, Escherichia coli xanthine guanine phosphoribosyl transferase (Ecogpt) gene and dehydrofolate reductase (dhfr) gene.

So as to produce the antibody for use in accordance with the invention, an appropriate expression system, for example eukaryotic cell or prokaryotic cell system can be used. The eukaryotic cell includes for example established animal cell lines such as mammalian cell lines, insect cell lines, fungal cells and yeast cells. The prokaryotic cell includes for example bacterial cells such as Escherichia coli cell.

Preferably, the antibody for use in accordance with the invention is expressed in mammalian cells, for example CHO, COS, myeloma, BHK, Vero, and HeLa cell.

The transformed host cell is cultured in vitro or in vivo to produce the intended antibody. The host cell may be cultured by known methods. As the culture medium, for example, DMEM, MEM, RPMI 1640 and IMDM can be used. Auxiliary serum fluid such as fetal calf serum (FCS) may also be used in combination.

The antibody expressed and generated as described above can be separated from such cells or host animals and can then be purified to homogeneity. The antibody for use in accordance with the invention can be separated and purified using an affinity column. A protein A column includes, for example, Hyper D, POROS, Sepharose F. F. (manufactured by Pharmacia). Additionally, any separation and purification methods generally used for protein may be employed in the invention. For example, chromatography columns other than affinity column, filter, ultrafiltration, salting-out, and dialysis may be used in combination to separate and purify the antibody (Antibodies A Laboratory Manual, Ed. Harlow, David Lane, Cold Spring Harbor Laboratory, 1988).

2. Detection of GPC3

Using the antibody against the N-terminal peptide of GPC3 in accordance with the invention, GPC3 in a test sample can be detected.

GPC3 to be detected using the antibody of the invention includes, but is not limited to, full-length GPC3 and fragments thereof. So as to detect GPC3 fragments, preferably, a fragment of the N-terminal peptide is detected.

The method for detecting the GPC3 protein in a test sample is not specifically limited. The GPC3 protein is preferably detected by an immunoassay method using the anti-GPC3 N-terminal peptide antibody. The immunoassaymethod includes, for example, radioimmunoassay, enzyme immunoassay, fluorescent immunoassay, luminescent immunoassay, immunoprecipitation method, immunonephelometry, western blot technique, immunostaining, and immunodiffusion method. Preferably, the immunoassay method is enzyme immunoassay. Particularly preferably, the immunoassay method is enzyme-linked immunosorbent assay (ELISA) (for example, sandwich ELISA). The immunoassay method such as ELISA as described above can be done by a person skilled in the art according to known methods.

General detection methods using the anti-GPC3 N-terminal peptide antibody to detect the GPC3 protein in a test sample involve, for example, immobilizing the anti-GPC3 N-terminal peptide antibody on a support, adding a test sample to the support for incubation to bind the GPC3 protein to the anti-GPC3 N-terminal peptide antibody, rinsing the support and detecting the GPC3 protein bound through the anti-GPC3 N-terminal peptide antibody to the support.

The support for use in accordance with the invention includes, for example, insoluble polysaccharides such as agarose and cellulose, synthetic resins such as silicone resin, polystyrene resin, polyacrylamide resin, nylon resin and polycarbonate resin, and insoluble supports such as glass. These supports can be used in the forms of beads and plates. In case of beads, a column packed with beads can be used. In case of plates, multi-well plate (for example, 96-well multi-well plate) and biosensor chip can be used. The anti-GPC3 N-terminal peptide antibody can be bound to the support by general methods such as chemical binding and phys ical adsorption. Such supports are commercially available.

The binding of the anti-GPC3 N-terminal peptide antibody to the GPC3 protein is generally done in buffers. For example, phosphate buffer, Tris buffer, citric acid buffer, borate salt buffer, and carbonate salt buffer may be used as a buffer. Incubation may be carried out under conditions commonly used, for example , 4 °C to ambient temperature for one hour to 24 hours. Rinsing after incubationmay be done using any solutions which do not inhibit the binding of the GPC3 protein to the anti-GPC3 N-terminal peptide antibody. For example, buffers containing surfactants such as Tween 20 may be used.

For the method for detecting the GPC3 protein in accordance with the invention, a control sample may be placed in addition to a test sample containing GPC3 protein to be detected. The control sample includes, for example, a negative control sample containing no GPC3 protein or a positive control sample containing the GPC3 protein. In this case, the GPC3 protein in the test sample can be detected by comparison with the results obtained using the negative control sample containing no GPC3 protein and the results obtained using the positive control sample containing the GPC3 protein. Additionally, a series of control samples having serially varied concentrations are prepared and the results of detection in the individual control samples are obtained in numerical figure to prepare a standard curve. Based on the standard curve, the GPC3 protein contained in the test sample can be determined quantitatively, based on the numerical figure about the test sample.

A preferable embodiment of the detection of the GPC3 protein bound through the anti-GPC3 N-terminal peptide antibody to the support includes a method using the anti-GPC3 N-terminal peptide antibody labeled with a labeling substance.

For example, a test sample is put in contact with the anti-GPC3 antibody immobilized on a support, which is then rinsed, to detect the GPC3 protein using a labeled antibody specifically recognizing the GPC3 protein.

In this case, the anti-GPC3 N-terminal peptide antibody immobilized on the support and anti-GPC3 N-terminal peptide C antibody labeled with a labeling substance may recognize the same epitope of the GPC3 molecule, but preferably recognize different epitopes.

The anti-GPC3 N-terminal peptide antibody can be labeled by generally known methods. Any labeling substances known to a person skilled in the art can be used, including for example fluorescent dye, enzyme, coenzyme, chemiluminescent substance and radioactive substance. Specific examples thereof include for example radioisotopes ($^{32}P$, $^{14}C$, $^{125}I$, $^{3}H$ and $^{131}I$), fluorescein, rhodamine, dansylchloride, umbelliferone, luciferase, peroxidase, alkaline phosphatase, β-galactosidase, β-glucosidase, horse radish peroxidase, glucoamylase, lysozyme, saccharide oxidase, microperoxidase, and biotin. Preferably, in the case that biotin is used as a labeling substance, avidin bound with enzymes such as alkaline phosphatase is further added after the addition of a biotin-labeled antibody. For binding the anti-GPC3 antibody with a labeling substance, any of the known methods such as glutaraldehyde method, maleimidemethod, pyridyl disulfide method and periodate method may be used.

Specifically, a solution containing the anti-GPC3 N-terminal peptide antibody is added to a support, such as a plate, to immobilize anti-GPC3 N-terminal peptide antibody. After rinsing the plate, the plate is blocked with for example BSA, so as to prevent non-specific protein binding. After rinsing again, a test sample is added to the plate. After incubation, the plate is rinsed, to which the labeled anti-GPC3 antibody is added. After appropriate incubation, the plate is rinsed and the labeled anti-GPC3 antibody remaining on the plate is detected. The detection can be done by methods known to a person skilled in the art. For example, in case of labeling with a radioactive substance, the detection can be done by a liquid scintillation or a RIA method. In case of labeling with an enzyme, a substrate for the respective enzyme is added to detect enzymatic substrate changes via for example color development by spectrophotometer. Specific examples of such substrate include 2,2-adinobis(3-ethylbenzothiazoline-6-sulfonic acid)diammonium salt (ABTS), 1,2-phenylenediamine (ortho-phenylenediamine), and 3,3',5,5'-tetramethylbenzidine (TME) . In case of labeling with a fluorescent substance, the fluorescent substance can be detected with fluorophotometer.

A particularly preferable embodiment of the method for detecting the GPC3 protein in accordance with the invention involves using anti-GPC3 N-terminal peptide antibody labeled with biotin and avidin.

Specifically,asolution containing anti-GPC3N-terminal peptide antibody is added to a support such as plate, to immobilize the anti-GPC3 N-terminal peptide antibody. After rinsing the plate, the antibody is blocked with for example BSA to prevent non-specific protein binding. After rinsing again, a test sample is added to the plate. After incubation, the plate is rinsed, and the biotin-labeled anti-GPC3 antibody is added. After appropriate incubation, the plate is rinsed, and avidin conjugated to an enzyme, such as alkaline phosphatase or peroxidase is added. After incubation, the plate is rinsed, a substrate corresponding to each enzyme conjugated to avidin is added, and the GPC3 protein is detected using an enzymatic substrate change as an indicator.

Another embodiment of the method for detecting the GPC3 protein in accordance with the invention involves using a primary antibody specifically recognizing the GPC3 protein and a secondary antibody specifically recognizing the primary antibody.

For example, a test sample is put in contact with the anti-GPC3 N-terminal peptide antibody immobilized on a support. After incubation, the support is rinsed and the GPC3 protein bound to the support after rinsing is detected using a primary anti-GPC3 antibody and a secondary antibody specifically recognizing the primary antibody. In this case, the secondary antibody is preferably labeled with a labeling substance.

Specifically,asolution containing anti-GPC3N-terminal peptide antibody is added to a support, such as plate, to immobilize the anti-GPC3 N-terminal peptide antibody. After rinsing the plate, the antibody is blocked with for example BSA to prevent non-specific protein binding. After rinsing again, a test sample is added to the plate. After incubation, the plate is rinsed and a primary anti-GPC3 antibody is added. After appropriate incubation, the plate is rinsed and a secondary antibody specifically recognizing the primary antibody is added. After appropriate incubation, the plate is rinsed and the secondary antibody remaining on the plate is detected. The detection of the secondary antibody can be done by the methods described above.

Still another embodiment of the method for detecting the GPC3 protein in accordance with the invention involves using an aggregation reaction. In this method, GPC3 can be detected using a carrier sensitized with the anti-GPC3 N-terminal peptide antibody. Any carriers may be used as the carrier to be sensitized with the antibody, as far as the carrier is insoluble and stable and does not undergo non-specific reaction. For example, latex particle, bentonite, collodion, kaolin and immobilized sheep erythrocyte may be used. Latex particle is preferably used. Latex particles include, for example, polystyrene latex particle, styrene-butadiene copolymer latex particle, and polyvinyltoluene latex particle. Polystyrene latex particle is preferably used. After the sensitized particle is mixed with a sample and agitated for a given period of time, GPC3 can be detected by observing the aggregation under naked eyes since the aggregation level of such particle is higher as the GPC3 antibody is contained at a higher concentration in the sample. Additionally, the turbidity due to the aggregation can be measured with spectrophotometer and the like, to detect GPC3.

Another embodiment of the method for detecting the GPC3 protein in accordance with the invention involves using a biosensor utilizing surface plasmon resonance phenomenon. The biosensor utilizing surface plasmon resonance phenomenon enables the observation of the protein-protein interaction as surface plasmon resonance signal on real time using a trace amount of protein without labeling. For example, the binding of the GPC3 protein to the anti-GPC3 N-terminal peptide antibody can be detected by us ing biosensors such as BIAcore (manufactured by Pharmacia) . Specifically, a test sample is put in contact with a sensor chip having the anti-GPC3 N-terminal peptide antibody immobilized thereon, and the GPC3 protein bound to the anti-GPC3 N-terminal peptide antibody is detected as the change of the resonance signal.

The detection methods in accordance with the invention may be automated using various automatic laboratory apparatuses, so that a large volume of samples can be tested at a time.

It is an objective of the invention to provide a diagnostic reagent or kit for detecting GPC3 protein in a test sample for cancer diagnosis. The diagnostic reagent or kit contains at least the anti-GPC3 N-terminal peptide antibody. In case that the diagnostic reagent or kit is based on EIA, a carrier for immobilizing the antibody may be contained, or the antibody may be preliminarily bound to a carrier. In case that the diagnostic reagent or kit is based on the aggregation method using carriers such as latex, the reagent of kit may contain a carrier having the antibody adsorbed thereon. Additionally, the kit may appropriately contain, for example, a blocking solution, a reaction solution, a reaction-terminating solution and reagents for treating sample.

3. Disruption of cancer cell using the anti-GPC3 C-terminal peptide antibody and cancer therapy using the same

(1) Determination of antibody activity

The antigen binding activity of the antibody for use in accordance with the invention may be assayed using known techniques (Antibodies A Laboratory Manual. Ed. Harlow, David Lane, Cold Spring Harbor Laboratory, 1988) and an activity of inhibiting the ligand-receptor binding thereof (Harada, A. et al., International Immunology (1993) 5, 681-690).

A method for assaying the antigen binding activity of the anti-GPC3.C-terminal peptide antibody for use in accordance with the invention includs ELISA (enzyme-linked immunosorbent assay), EIA (enzyme immunoassay), RIA (radioimmunoassay) and fluorescent antibody method. In enzyme immunoassay, a sample containing the anti-GPC3 C-terminal peptide antibody, for example a culture supernatant of a cell producing the anti-GPC3 C-terminal peptide antibody or the purified antibody is added to a plate coated with the GPC3 C-terminal peptide. A secondary antibody labeled with an enzyme such as alkali phosphatase is added and the plate is incubated and rinsed, then an enzyme substrate such as p-nitrophenylphosphoric acid is added to measure the absorbance and assess the antigen binding activity.

So as to determine the activity of the antibody for use in accordance with the invention, the neutralization activity of the anti-GPC3 C-terminal peptide antibody is measured.

(2) Cytotoxicity

For therapeutic purpose, the antibody for use in accordance with the invention preferably has the ADCC activity or the CDC activity as cytotoxicity.

The ADCC activity can be assayed by mixing an effector cell, a target cell and the anti-GPC3 C-terminal peptide antibody together and examining the ADCC level. As the effector cell, cell such as mouse splenocyte and mononuclear cell separated from human peripheral blood or bone marrow can be utilized. As the target cell, a human cell line such as human hepatoma line HuH-7 can be used. The target cells are preliminarily labeled with $^{51}$Cr and incubated with the anti-GPC3 C-terminal peptide antibody, then effector cells at an appropriate ratio is added to the target cells and incubated. After incubation, the supernatant is collected to count the radioactivity in the supernatant, to assay the ADCC activity.

Further, the CDC activity can be assayed by mixing the labeled target cell described above with the anti-GPC3 C-terminal peptide antibody, subsequently adding complement, and counting the radioactivity in the supernatant after incubation.

The Fc moiety is needed for the antibody to exert the cytotoxicity. In case that the inhibitor of cell proliferation in accordance with the invention utilizes the cytotoxicity of the antibody, thus, the anti-GPC3 C-terminal peptide antibody for use in accordance with the invention preferably contains the Fc moiety.

(3) Cell disruption

The anti-GPC3 C-terminal peptide antibody of the invention may also be used for cell disruption, particularly the disruption of cancer cell. Further, the anti-GPC3 C-terminal peptide antibody of the invention can be used as an anticancer agent. Cancers to be therapeutically treated and prevented by the antibody of the invention include, but are not limited to, hepatoma, lung cancer, colon cancer, breast cancer, prostate cancer, pancreatic cancer and lymphoma, preferably Hepatoma.

(4) Administration method and pharmaceutical formulation

The cell disrupting agent or anticancer agent in accordance with the invention is used for the purpose of therapeutically treating or ameliorating diseases caused by abnormal cell growth, particularly cancer.
The effective dose is selected within a range of 0.001 mg to 1,000 mg per 1 kg body weight. Also the effective dose is selected within a range of 0. 01 mg to 100, 000 mg/body weight per patient. However, the dose of the therapeutic agents containing the anti-GPC3 C-terminal peptide antibody of the invention are not limited to the above doses.
The timing for administering the therapeutic agent of the invention is either before or after the onset of clinical symptoms of the diseases.
The therapeutic agent comprising the anti-GPC3 C-terminal-peptide antibody in accordance with the invention as an active component can be formulated by a conventional method (Remington's Pharmaceutical Science, latest edition, Mark Publishing Company, Easton, USA), and may also contain pharmaceutically acceptable carriers and additives.
Examples of such carriers and pharmaceutical additives include water, pharmaceutically acceptable organic solvents, collagen, polyvinyl alcohol, polyvinyl pyrrolidone, carboxyvinyl polymer, carboxymethyl cellulose sodium, polyacrylate sodium, sodium alginate, water-soluble dextran, carboxymethyl starch sodium, pectin, methyl cellulose, ethyl cellulose, gum xanthan, gum arabic, casein, agar, polyethylene glycol, diglycerin, glycerin, propylene glycol, vaseline, paraffin, stearyl alcohol, stearic acid, human serum albumin (HSA), mannitol, sorbitol, lactose and surfactants acceptable as pharmaceutical additives.
In practice, an additive or a combination thereof is selected depending on the dosage form of the therapeutic agent of the invention. However, the additive is not limited to those described above. In case that the therapeutic agent is to be used in an injection formulation, the purified anti-GPC3 C-terminal peptide antibody of the invention is dissolved in a solvent, such as physiological saline, buffers, and glucose solution, and adsorption preventing agents such as Tween 80, Tween 20, gelatin and human serum albumin is added. Alternatively, the therapeutic agent is provided in a freeze-dried form as a dosage form to be dissolved and reconstituted prior to use. As excipients for freeze-drying, for example, sugar alcohols such as mannitol and glucose and sugars may be used.

Brief Description of the Drawings

Fig. 1 shows bar graphs depicting the results of the analysis of GPC3 mRNA expression using Gene Chip, where Fig. 1A depicts GPC3 expression and Fig. 1B depicts the expression of alpha-fetoprotein (AFP) . NL, CH, LC, WD, MD and PD on the holizontal axis represent normal liver, inflammatory lesion of hepatitis, lesion of liver cirrhosis, well-differentiated cancer, moderately differentiated cancer and poorly differentiated cancer, respectively.
Fig. 2 shows images of purified soluble GPC3 of heparan sulfate adduct type and the GPC3 core protein, as stained with CBB.
Fig. 3 shows bar graphs depicting the expression of the GPC3 gene in human hepatoma.
Fig. 4 shows the results of western blotting of the soluble form of the core protein using the anti-GPC3 antibody.
Fig. 5 shows the principle of sandwich ELISA using the anti-GPC3 antibody.
Fig. 6 is a graph of the standard curve for the GPC3 sandwich ELISA using M6B1 and M18D4.
Fig. 7 is a schematic view of the GPC3 structure.
Fig. 8 shows combinations of the anti-GPC3 antibodies employed in ELISA.
Fig. 9 is a graph of the standard curve for the GPC3 sandwich ELISA system using various combinations of the anti-GPC3 antibodies.
Fig. 10 shows the assay results of the ADCC activity of the anti-GPC3 antibody.
Fig. 11 shows the assay results of the CDC activity of the anti-GPC3 antibody.

Best Mode for Carrying out the Invention

The invention is now specifically described in the following Examples. However, the invention is not limited by the Examples.
In the Examples described in this specification, the following materials were used.
As expression vectors of the soluble form of GPC3 and the soluble form of the GPC3 core protein, pCXND2 and pCXND3 prepared by integrating the DHFR gene and the neomycin-resistant gene in pCAGGS were used.
DXB11 was purchased from ATCC. For culturing, 5 % FBS (GIBCO BRL CAT# 10099-141, Lot# AO275242/Minimum Essential Medium Alpha medium (αMEM (+)) (GIBCO BRL CAT# 12571-071) /1 % Penicillin-Streptomycin (GIBCO BRL CAT# 15140-122) was used. For selection of stable cell line of DXB11 expressing each protein, 500 μg/mL Geneticin

(GIBCO BRL CAT# 10131-027)/5 % FBS/α MEM without ribonucleotides and deoxyribonucleotides (GIBCO BRL CAT# 12561-056) (αMEM(-)) /PS was used alone or with supplemented with MTX to a final concentration of 25 nM.

HepG2 was purchased from ATCC and maintained in 10 % FBS/Dulbecco's modified Eagle medium (DMEM) (GIBCO BRL CAT# 11995-065)/PS.

The hybridoma was maintained in 10 % FBS/RPMI1640/1 × HAT media supplement (SIGMA CAT# H-0262) /0.5 × BM-Condimed H1 Hybridoma cloning supplement (Roche CAT# 1088947).

Example 1

Cloning and expression analysis of human GPC3 (GPC3) cDNA Cloning of full-length cDNA encoding human glypican 3 (GPC3 hereinafter)

The full-length cDNA encoding human GPC3 was amplified by PCR, using as a template a first strand cDNA prepared from a colon cancer cell line Caco2 by a general method and Advantage 2 kit (Clontech Cat. No. 8430-1). Specifically, 50 μl of a reaction solution containing Caco2-derived cDNA of 2 μl, 1 μl of a sense primer (SEQ ID NO: 1), 1 μl of an antisense primer (SEQ ID NO: 2), 5 μl of Advantage2 10 × PCR buffer, 8 μl of dNTP mix (1.25 mM) and 1.0 μl of Advantage polymerase Mix was subjected to 35 cycles of 94 °C for one minute, 63 °C for 30 seconds and 68 °C for 3 minutes. The amplified product from the PCR (inserted in TA vector pGEM-T easy using pGEM-T Easy Vector System I (Promega Cat No. A1360)) was sequenced using ABI3100 DNA sequencer to confirm that cDNA encoding the full-length human GPC3 was isolated. The sequence represented by SEQ ID NO: 3 indicates the nucleotide sequence of the human GPC3 gene, while the sequence represented by SEQ ID NO: 4 indicates the amino acid sequence of human GPC3 protein.

SEQ ID NO: 1: GATATC-ATGGCCGGGACCGTGCGCACCGCGT
SEQ ID NO: 2: GCTAGC-TCAGTGCACCAGGAAGAAGAAGCAC

Expression Analysis of human GPC3 mRNA using GeneChip

mRNA expression was analyzed in 24 cases with hepatoma lesions (well-differentiated cancer: WD; moderately differentiated cancer: MD; poorly differentiated cancer: PD), 16 hepatoma cases with non-cancer lesions (hepatitis lesion: CH, cirrhosis lesion : LC), 8 cases with normal liver: NL (informed consent acquired; available from Tokyo University, School of Medicine and Saitama Cancer Center), using GeneChip™ UG-95A Target (Affymetrix). Specifically, total RNA was prepared using ISOGEN (Nippon Gene) from the individual tissues, from which 15 μg each of total RNA was used for gene expression analysis according to the Expression Analysis Technical Manual (Affymetrix).

As shown in Fig.1, the mRNA expression level of human GPC3 gene (Probe Set ID: 39350_at) was apparently higher in many of the cases compared with the expression in normal liver tissue, despite the differentiation stages of hepatoma. Furthermore, comparison was made with the mRNA expression of alpha-fetoprotein (Probe Set ID: 40114_at) most commonly used as a diagnostic marker of hepatoma currently. It was shown that even in well-differentiated cancer showing almost no such mRNA expression of alpha-fetoprotein, sufficiently enhanced mRNA expression of GPC3 was observed, and that the ratio of the activation of the mRNA expression of GPC3 was higher. Thus, it is considered that GPC3 detection is useful as a diagnostic method of hepatoma at an early stage.

Example 2

Preparation of anti-GPC3 antibody

Preparation of the soluble form of human GPC3

As a material for preparing anti-GPC3 antibody, the soluble form of the GPC3 protein lacking the hydrophobic region on the C-terminal side was prepared.

Using a plasmid DNA containing the complete full-length human GPC3 cDNA supplied from Tokyo University, Advanced Technology Institute, a plasmid DNA for expressing the soluble form of the GPC3 cDNA was constructed. PCR was conducted using a downstream primer (5'-ATA GAA TTC CAC CAT GGC CGG GAC CGT GCG C-3') (SEQ ID NO: 5) designed to remove the hydrophobic region on the C-terminal side (564-580 amino acid), and an upstream primer (5'-ATA GGA TCC CTT CAG CGG GGA ATG AAC GTT C-3') (SEQ ID NO.6) with the EcoRI recognition sequence and the Kozak's sequence having been added. The resulting PCR fragment (1711 bp) was cloned in pCXND2-Flag: The prepared expression plasmid DNA was introduced in a CHO cell,line DXB11. Selection with 500 μg/mL Geneticin resulted in a CHO line highly expressing the soluble form of GPC3.

Using a 1700-cm$^2$ roller bottle, the CHO line highly expressing the soluble form of GPC3 was cultured at a large scale, and the culture supernatant was collected for purification. The culture supernatant was applied to DEAE Sepharose Fast Flow (Amersham CAT# 17-0709-01), washed, and eluted with a buffer containing 500 mM NaCl. Subsequently, the product was affinity purified using Anti-Flag M2 agarose affinity gel (SIGMA CAT# A-2220) and eluted with 200 μg/mL Flag peptide. After concentration with Centriprep-10 (Millipore Cat# 4304), the Flag peptide was removed by gel filtration with Superdex 200 HR 10/30 (Amersham CAT# 17-1088-01). Finally, the product was concentrated using DEAE Sepharose Fast Flow column, and eluted with PBS (containing 500 mM NaCl) containing no Tween 20 for replacement of the buffer.

Preparation of the soluble form of human GPC3 core protein

Using the wild type human GPC3 cDNA as template, cDNA was prepared by assembly PCR, where Ser 495 and Ser 509 were substituted with Ala. A primer was designed in such a fashion that His tag might be added to the C terminus. The resulting cDNA was cloned in pCXND3 vector. The prepared expression plasmid DNA was introduced in a DXB11 line, followed by selection with 500 μg/mL Geneticin, to obtain the CHO line highly expressing the soluble form of the GPC3 core protein.
A large scale cultivation was done with a 1700-cm$^2$ roller bottle, and.the culture supernatant was collected for purification. The supernatant was applied to Q sepharose Fast Flow (Amersham CAT# 17-0510-01), washed, and eluted with a phosphate buffer containing 500 mM NaCl. Subsequently, the product was affinity purified using Chelating Sepharose Fast Flow (Amersham CAT# 17-0575-01), and eluted with a gradient of 10-150 mM imidazole. Finally, the product was concentrated with Q sepharose Fast Flow and eluted with a phosphate buffer containing 500 mM NaCl.
SDS polyacrylamide gel electrophoresis showed a smear-like band of 50 to 300 kDa and a band of about 40 kDa. Fig.2 shows the results of the electrophoresis. GPC3 is a proteoglycan of 69 kDa and with a heparan sulfate-addition sequence at the C terminus. It was considered that the smear-like band corresponds to GPC3 modified with heparan sulfate. The results of amino acid sequencing indicated that the band of about 40 kDa had an origin in the N-terminal fragment. Thus, it was anticipated that GPC3 was more or less cleaved.
So as to remove antibodies against heparan sulfate in the following screening for hybridoma, the soluble form of the GPC3 core protein where a heparan sulfate-addition signal sequence Ser 495 and Ser 509 were substituted with Ala. CHO cell line highly expressing the protein was prepared as above, and the culture supernatant was affinity purified utilizing the His-tag. SDS polyacrylamide gel electrophoresis showed three bands of 70 kDa, 40 kDa and 30 kDa. Amino acid sequencing indicated that the band of 30 kDa was the C-terminal fragment of GPC3. The C-terminal fragment starts from serine 359 or from valine 375. Thus, it was anticipated that GPC3 received some enzymatic cleavage. The reason why the band of 30 kDa was not observed in the GPC3 of heparan sulfate-added type was that the fragment formed the smear-like band due to the addition of heparan sulfate. It is a novel finding that GPC3 receives enzymatic cleavage at a specific amino acid sequence, but the biological meaning thereof has not yet been elucidated.
The inventors made an assumption on the basis of the results that GPC3 on the membrane even in hepatoma patients would be cleaved and secreted as the soluble form in blood. Compared with AFP as a hepatoma marker, the expression of the gene of GPC3 was found higher in hepatoma patients at earlier stages (Fig. 1) . So as to examine the possibility as a novel tumor marker with higher clinical utility than that of AFP, an anti-GPC3 antibody was prepared to construct a sandwich ELISA system as described in Example 2 or below.

Preparation of anti-GPC3 antibody

Because the homology of human GPC3 with mouse GPC3 is as high as 94 % at the amino acid levels, it was considered that it might be difficult to obtain the anti-GPC3 antibody by the immunization of normal mouse with human GPC3. Thus, MRL/lpr mouse with autoimmune disease was used as an animal to be immunized. Five MRL/lpr mice (CRL) were immunized with the soluble form of GPC3. For the first immunization, the immunogen protein was adjusted to 100 μg/animal and was then emulsified using FCA (Freund's complete adjuvant (H37 Ra), Difco (3113-60), Becton Dickinson (cat# 231131)), which was then subcutaneously administered to the mice. Two weeks later, the protein was adjusted to 50 μg/animal and emulsified with FIA (Freund's incomplete adjuvant, Difco (0639-60), Becton Dickinson (cat# 263910)) for subcutaneous administration to the mice. At one week interval since then, booster was carried out in total of 5 times. For final booster, the protein was diluted with PBS to 50 μg/animal, which was administered in the caudal vein. By ELISA using an immunoplate coated with the GPC3 core protein, it was confirmed that the serum antibody titer against GPC3 was saturated. A mouse myeloma cell P3U1 and mouse splenocyte were mixed together to allow for cell fusion in the presence of PEG1500 (Roche Diagnostics, cat# 783641). The resulting mixture was inoculated in a 96-well culture plate. From the next day, hybridoma was selected with the HAT medium, the culture supernatant was screened by ELISA. Positive clones were subjected to monocloning by limited dilution method. The resulted monoclone was cultured at an enlarged scale and the culture supernatant was collected. The screening by ELISA was done using the binding activity to the GPC3 core protein as a marker to obtain six clones of an anti-GPC3 antibody with a strong binding potency.

The antibody was purified using Hi Trap Protein G HP (Amersham CAT# 17-0404-01). The supernatant from the hybridoma culture was applied directly to a column, washed with a binding buffer (20 mM sodiumphosphate, pH 7.0) and eluted with an elution buffer (0.1 M glycine-HCl, pH 2.7). The eluate was collected into a tube containing a neutralization buffer (1 M Tris-HCl, pH 9. 0) for immediate neutralization. After antibody fractions were pooled, the resulting pool was dialyzed against 0.05 % Tween 20/PBS overnight and for a whole day for buffer replacement. $NaN_3$ was added to the purified antibody to 0.02 %. The antibody was stored at 4 °C.

Analysis of anti-GPC3 antibody

The antibody concentration was assayed by mouse IgG sandwich ELISA using goat anti-mouse IgG (gamma) (ZYMED CAT# 62-6600) and alkali phosphatase-goat anti-mouse IgG (gamma) (ZYMED CAT# 62-6622), along with a commercially available purifiedmouse IgG1 antibody (ZYMED CAT# 02-6100) as a standard.

The isotyping of the anti-GPC3 antibody was done with ImmunoPure Monoclonal Antibody Isotyping Kit II (PIERCE CAT# 37502) by the method according to the attached manual. The results of the isotyping indicated that all of the antibodies were of IgGl type.

By western blotting using the GPC3 core protein, the epitopes of.the anti-GPC3 antibody were classified. The soluble form of the GPC3 core protein was applied to 10 % SDS-PAGE mini (TEFCO CAT# 01-075) at 100 ng/lane for electrophoresis (60 V for 30 min; 120 V for 90 min), and subsequently transferred on Immobilon-P (Millipore CAT# IPVH R85 10) using Trans-Blot SD Semi-Dry Electrophoretic Transfer Cell (BIO-RAD) (15 V for 60 min). After the membrane was gently rinsed with TBS-T (0.05% Tween 20, TBS), the membrane was shaken with 5 % skim milk-containing TBS-T for one hour (at ambient temperature) or overnight (at 4 °C) . After shaking with TBS-T for about 10 minutes, each anti-GPC3 antibody diluted with 1 % skim milk-containing TBS-T to 0.1 to 10 μg/ml was added for one-hour with shaking. The membrane was rinsed with TBS-T (10 minutes × three times) and shaken with HRP-anti-mouse IgG antibody (Amersham CAT# NA 931) diluted to 1.1000 with 1 % skim milk-containing TBS-T for one hour, and rinsed with TBS-T (10 minutes × three times) . ECL-Plus (Amersham RPN 2132) was used for chromogenic reaction. Hyperfilm ECL (Amersham CAT# RPN 2103K) was used for detection. Fig. 4 shows the results of the western blotting analysis. For the classification, it was determined that the antibody reacting with the band of 40 kDa has an epitope at the N terminus, while the antibody reacting with the band of 30 kDa has an epitope at the C terminus. As antibodies recognizing the N-terminal side, M6B1, M18D4, and M19B11 were obtained. As antibodies recognizing the C-terminal side, M3C11, M13B3, and M3B8 were obtained. The results of the analysis using BIACORE indicated that the KD values of the individual antibodies were in the range of from 0.2 to 17.6 nM.

Example 3

Detection of the secreted form of GPC3

Mouse xenograft model

3,000,000 human hepatoma HepG2 cells were transplanted under the abdominal skin in 6-weeks female SCID mice (Fox CHASE C. B-17/Icr-scidJcl, JapanClair) andnudemice (BALB/cAJcl-nu, Japan Clair) . 53 days later when tumor was sufficiently formed, whole blood was drawn out from the posterior cava of HepG2-transplanted SCID mice #1, 3, and 4. Plasma was prepared in the presence of EDTA-2Na and aprotinin (Nipro Neotube vacuum blood tube, NIPRO, NT-EA0205) and stored at -20 °C until assay date. In the case of the HepG2-transplanted SCID mouse #2, whole blood was taken 62 days after HepG2 transplantation. In the case of the HepG2-transplanted nude mice #1 and #2, whole blood was taken 66 days after HepG2 transplantation. As a control, plasma was prepared from normal SCID mouse of the same age by the same procedures.

Sandwich ELISA

So as to detect the secreted form of GPC3 in blood, a sandwich ELLSA system of GPC3 was constructed. M6B1 was used as an antibody to be coated in a 96-well plate. M18D4 labeled with biotin was used as an antibody detecting GPC3 bound to M6B1. For chromogenic reaction, AMPAK of DAKO was used for achieving high detection sensitivity.

A 96-well immunoplate was coated with the anti-GPC3 antibody diluted with a coating buffer (0.1 M $NaHCO_3$, pH 9.6, 0.02 w/v % $NaN_3$) to obtain a concentration of 10 μg/mL, and incubated at 4 °C overnight. On the next day, the plate was rinsed three times with 300 μl/well of rinse buffer (0.05 v/v %, Tween 20, PBS) and 200 μl of dilution buffer (50 mM Tris-HCl, pH 8.1, 1 mM $MgCl_2$, 150 mM NaCl, 0.05 v/v % Tween 20, 0.02 w/v % NaN 3, 1 w/v % BSA) was added for blocking. After storage for several hours at ambient temperature or at 4 °C overnight, mouse plasma or the culture supernatant appropriately diluted with a dilution buffer was added and incubated at ambient temperature for one hour. After rinsing with RB at 300 μl/well three times, the biotin-labeled anti-GPC3 antibody diluted with a dilution buffer to 10 μg/mL was added, and incubated at ambient temperature for one hour. After rinsing with RB at 300 μl/well three times, AP-streptoavidin (ZYMED) diluted to 1/1000 with a dilution buffer was added, and incubated at ambient temperature for one hour. After rinsing with the rinse buffer at 300 μl/well five times, AMPAK (DAKO CAT# K6200) was added for chromogenic reaction according to the attached protocol, and the absorbance was measured with a microplate reader.

For biotinylation of the antibody, Biotin Labeling Kit (CAT# 1 418 165) of Roche was used. A spreadsheet software GlaphPad PRISM (GlaphPad software Inc. ver. 3.0) was used to calculate the concentration of the soluble form of GPC3 in a sample. Fig.5 shows the principle of the sandwich ELISA in this Example.

Using the purified soluble form of GPC3, a standard curve was prepared. Consequently, a system with a detection limit of several nanogams/mL could be constructed. Fig.6 shows a standard curve for the GPC3 sandwich ELISA using M6B1 and M18D4. Using the system, an attempt was made to detect the secreted form of GPC3 in the culture supernatant of HepG2 and the serum of a mouse transplanted with human hepatoma HepG2 . The secreted form of GPC3 was detected in the culture supernatant of HepG2 and the serum of the mouse transplanted with human hepatoma HepG2, while the secreted form of GPC3 was below the detection limit in the control culture medium and the control mouse serum. On a concentration basis of the purified soluble form of GPC3, the soluble form of GPC3 was at 1.2 μg/mL in the culture supernatant of HepG2 and at 23 to 90 ng/mL in the serum of the mouse (Table 1).

Table 1

| Assay of the secreted form of GPC3 in the plasma of a mouse transplanted with HepG2 (ng/mL) | | | | | | |
|---|---|---|---|---|---|---|
| | Tumor volume ($mm^3$) | M6B01 (N)-M 1BD4 (N) | M19B11 (N)-M18D4(N) | M6B1 (N)-BioM3C11 (C) | M13B3(C)-Bi oM18D4(N) | M13B3(C)-Bi oM3B8(C) |
| Culture supernatant of HepG2 | | 1190 | 1736 | 224 | 234 | <1 |
| HepG2-transplanted SCID mouse #1 | 2022 | 65.4 | 76.9 | <10 | <10 | <10 |
| HepG2-transplanted SCID mouse #2 | 1706 | 71.7 | 94.8 | <10 | <10 | <10 |
| HepG2-transplanted SCID mouse #3 | 2257 | 90.3 | 113.9 | <10 | <10 | <10 |
| HepG2-transplanted SCID mouse #4 | 2081 | 87.3 | 107.3 | <10, | 15.0 | <10 |
| HepG2-transplanted nude mouse #1 | 1994 | 58.7 | 53.6 | 19.7 | 35.5 | 102.2 |
| HepG2-transplanted nude mouse #2 | 190 & 549 | 22.9 | 33.6 | <10 | 11.5 | 40.6 |
| Normal SCID mouse #1 | 0 | <10 | <10 | <10 | <10 | <10 |

(continued)

| Assay of the secreted form of GPC3 in the plasma of a mouse transplanted with HepG2 (ng/mL) | | | | | | |
|---|---|---|---|---|---|---|
| | Tumor volume ($mm^3$) | M6B01 (N)-M 1BD4 (N) | M19B11 (N)-M18D4(N) | M6B1 (N)-BioM3C11 (C) | M13B3(C)-BioM18D4(N) | M13B3(C)-BioM3B8(C) |
| Normal SCID mouse #2 | 0 | <10 | <10 | <10 | <10 | <10 |
| Normal SCID mouse #3 | 0 | <10 | <10 | <10 | <10 | <10 |

Structure of secreted form of GPC3

It was examined whether or not the blood-secreted GPC3 has the structure of the N-terminal fragment as preliminarily assumed. In case that the secreted form of GPC3 was the N-terminal fragment, it is considered that the secreted form of GPC3 will not be detected by sandwich ELISA with a combination of an antibody recognizing the N terminus and an antibody recognizing the C terminus. Using three types of each antibody recognizing the N-terminal fragment and each'antibody recognizing the C-terminal fragment, sandwich ELISA systems with various combinations were constructed. Fig.7 shows the structure of the secreted form of GPC3 and Fig.8 shows combinations of the antibodies. Fig.9 shows a standard curve of the sandwich ELISA. Table 1 shows the assay results. As shown in Table 1, the secreted form of GPC3 was detected at higher values in the culture supernatant of HepG2 and the serum of a mouse transplanted with human hepatoma HepG2 with combinations of antibodies recognizing the N-terminal fragment, while it was detected below the detection limit in many samples from the mice with the systems containing antibodies recognizing the C-terminal fragment. Thus, it was anticipated that the secreted form of GPC3 dominantly comprises the N-terminal fragment. Accordingly, it was suggested that the blood-secreted GPC3 was possibly detected at a high sensitivity by using an antibody against the amino acid sequence comprising the amino acid residue 1 to the amino acid residue 374 of GPC3.

Example 4

Preparation of anti-GPC3 mouse-human chimera antibody

Using total RNA extracted from a hybridoma producing an antibody capable of binding to human GPC3 (human GPC3-antibody recognizing C-terminus: M3C11, M1E07; human GPC3-antibody recognizing N terminus: M19B11, M18D04, M5B09, M10D02), the cDNA of variable region of the antibody was amplified by RT-PCR. The total RNA was extracted from the hybridoma of $1 \times 10^7$ cells, using RNeasy Plant Mini Kits (manufactured by QIAGEN). Using 1 $\mu$g of the total RNA and also using SMART RACE cDNAAmplification Kit (manufactured by CLONTECH), a synthetic oligonucleotide MHC-IgG1 (SEQ ID NO:7) complementary to the mouse IgG1 constant region sequence or a synthetic oligonucleotide kappa (SEQ ID NO:8) complementary to the nucleotide sequence of the mouse $\kappa$ chain constant region, a 5'-terminal fragment of the gene was amplified. The reverse-transcription was done at 42 °C for one hour and 30 minutes. 50 $\mu$l of the PCR solution contained 5 $\mu$l of 10 $\times$ Advantage 2 PCR Buffer, 5 $\mu$l of 10 $\times$ Universal Primer A Mix, 0.2 mM dNTPs (dATP, dGTP, dCTP, dTTP), 1 $\mu$l of Advantage 2 Polymerase Mix (all manufactured by CLONTECH), 2.5 $\mu$l of the reverse-transcription product, and 10 pmole of the synthetic oligonucleotide MHC-IgGl or kappa. After the initial temperature at 94 °C for 30 seconds, a cycle of 94 °C for 5 seconds and 72 °C for 3 minutes was repeated five times; a cycle of 94 °C for 5 seconds, 70 °C for 10 seconds and 72 °C for 3 minutes was repeated five times; and a cycle of 94 °C for 5 seconds, 68 °C for 10 seconds and 72 °C for 3 minutes was repeated 25 times. Finally, the reaction product was heated at 72 °C for 7 minutes. After the individual PCR products were purified from agarose gel using QIAquick Gel Extraction Kit (manufactured by QIAGEN), the products were cloned in pGEM-T Easy vector (manufactured by Promega), and the nucleotide sequence was determined.

Then, the sequences of the variable regions of the H chain and L chain were linked to the constant regions of the human H chain and L chain. PCR was done using a synthetic oligonucleotide complementary to the 5'-terminal nucleotide sequence of the H chain variable region of each antibody and having the Kozak's sequence and a synthetic oligonucleotide complementary to the 3'-terminal nucleotide sequence and having an NheI site. The resulting PCR products were cloned in a pB-CH vector with the human IgG1 constant region inserted in pBluescript KS+ vector (manufactured by TOYOBO). The mouse H chain variable region and the human H chain ($\gamma$1 chain) constant region are liked together via the NheI site. The prepared H chain gene fragment was cloned in an expression vector pCXND3. The scheme of the construction of the vector pCXND3 is described below. So as to divide the gene encoding the antibody H chain and the vector sequence from DHFR-ΔE-rvH-PM1-f (see WO 92/19759), the vector was digested at the restriction enzyme EcoRI/SmaI sites to recover only the vector sequence. Subsequently, the vector sequence was cloned in EcoRI-NotI-BamHI adaptor (manufactured by Takara Shuzo Co., Ltd.). This vector was designated as pCHO1. A region from pCHO1 expressing the DHFR gene was cloned in pCXN at the restriction enzyme HindIII site (Niwa et al., Gene 1991: 108: 193-200). The resulting vector was designated as pCXND3. The nucleotide sequences of the H chains of the anti-GPC3 mouse-human chimera antibodies (M3C11, M1E07, M19B11, M18D04) contained in each plasmid are shown as SEQ ID NOS:.9, 11, 13 and 15, respectively. The amino acid sequences thereof are shown as SEQ ID NOS: 10, 12, 14, and 16, respectively. Additionally, PCR was done using a synthetic oligonucleotide complementary to the 5'-terminal nucleotide sequence of the L chain variable region of each antibody and having the Kozak's sequence and a synthetic oligonucleotide complementary to the 3'-terminal nucleotide sequence and having a BsiWI site. The resulting PCR products were cloned in a pB-CL vector, where the human kappa chain constant region was preliminarily inserted in pBluescript KS+ vector (manufactured by TOYOBO). The human L chain variable region and the constant region were linked together via the BsiWI site. The prepared L chain gene fragment was cloned in an expression vector pUCAG. The vector pUCAG is a vector prepared by digesting pCXN ( Niwa et al., Gene 1991: 108: 193-200) with restriction enzyme BamHI to obtain a 2.6-kbp fragment, which is then cloned into the restriction enzyme BamHI site of pUC19 vector (manufactured by TOYOBO). The nucleotide sequences of the L chains of the anti-GPC3mouse-human chimera antibodies (M3C11, M1E07,M19B11, M18D04) contained in each plasmid are shown as SEQ ID NOS: 17, 19, 21 and 23, respectively. The amino acid sequences thereof are shown as SEQ ID NOS: 18, 20, 22 and 24, respectively.

So as to prepare an expression vector of the anti-GPC3 mouse-human chimera antibody, a gene fragment obtained by digesting the pUCAG vector having the L chain gene fragment inserted therein with restriction enzyme HindIII (manufactured by Takara Shuzo Co., Ltd.) was cloned into the restriction enzyme HindIII cleavage site of pCXND3 having the H chain gene inserted therein. The plasmid will express the neomycin-resistant gene, the DHFR gene and the anti-GPC3 mouse-human chimera antibody gene in animal cells.

A CHO-based cell line for stable expression (DG44 line) was prepared as follows. The gene was introduced by electroporation method using Gene PulserII (manufactured by Bio Rad). 25 μg of each expression vector of the anti-GPC3 mouse-human chimera antibody and 0.75 ml of CHO cells (1 x $10^7$ cells/ml) suspended in PBS were mixed together, and cooled on ice for 10 minutes, which was then transferred into a cuvette and received a pulse at 1.5 kV and 25 μFD. After a recovery time at ambient temperature for 10 minutes, the cells treated by the electroporation were suspended in 40 mL of a CHO-S-SFMII culture medium (manufactured by Invitrogen) containing 1 × HT supplement (manufactured by Invitrogen). A 50-fold dilution was prepared using the same culture medium, and added at 100 μl/well in a 96-well culture plate. After culturing in a $CO_2$ incubator (5 % $CO_2$) for 24 hours, Geneticin (manufactured by Invitrogen) was added to 0.5 mg/mL, and continued cultivation for 2 weeks. The IgG in the culture supernatant from the wells of colonies of a Geneticin resistance transformant cell was assayed by the following concentration assay method. A cell line with high productivity was expanded at an enlarged scale. The cell line stably expressing the anti-GPC3 mouse-human chimera_antibody was cultured in a large-scale culturing and the culture supernatant was collected.

The IgG concentration in the culture supernatant was assayed by human IgG sandwich ELISA using Goat Anti-human IgG (manufactured by BIOSORCE) and Goat Anti-human IgG alkaline phosphatase conjugated (manufactured by BIOSORCE) and compared with the commercially available purified human IgG (manufactured by Cappel).

Each anti-GPC3 mouse-human chimera antibody was purified using Hi Trap Protein G HP (manufactured by Amersham). A culture supernatant of a CHO cell line producing the anti-GPC3 mouse-human chimera antibody was directly applied to a column and eluted with elution buffer (0.1 M glycine-HC1, pH 2.7). Eluate was collected into a tube containing a neutralization buffer (1 M Tris-HC1, pH 9.0) for immediate neutralization. Antibody fractions were pooled and dialyzed against 0.05% Tween 20/PBS overnight and for a whole day to replace the buffer. $NaN_3$ was added to the purified antibody to 0.02 % and stored at 4 °C.

Example 5

Preparation of a CHO cell line stably expressing the full length GPC3

Human .GPC3 cDNA was obtained by digesting pGEM-T Easy vector with the full-length human GPC3 cDNA cloned therein with restriction enzyme EcoRI (manufactured by Takara Shuzo Co., Ltd.) and cloned in an expression vector pCOS2. The scheme of the construction of the vector pCOS2 is described below. So as to divide the gene of the antibody H chain of DHFR-ΔE-rvH-PM1-f (see WO 92/19759) from the vector, the vector was digested at the restriction enzyme EcoRI/SmaI sites, to recover only the vector sequence. Subsequently, the vector sequence was cloned in EcoRI-NotI-BamHI adaptor (manufactured by Takara Shuzo Co., Ltd.). This vector was designated as pCHO1. A region from pCHO1 expressing the DHFR gene was removed, into which the sequence of the neomycin resistant gene in HEF-VH-gγ1 (Sato et al., Mol. Immunol. 1994: 31: 371-381) was inserted. The vector was designated as pCOS2.

A cell line stably expressing the full-length human GPC3 was prepared as follows. 10 μl of the full-length human GPC3 gene-expressing vector and 60 μl of SuperFect (manufactured by QIAGEN) were mixed together, to form a complex, which was then added to a CHO cell line DXB11 to introduce the gene. After culturing in a $CO_2$ incubator (5 % $CO_2$) for 24 hours, αMEM (manufactured by GIBCO BRL) containing Geneticin (manufactured by Invitrogen) to a final concentration of 0.5 mg/mL and 10 % FBS (manufactured by GIBCO BRL) was used to start selection. The resulting Geneticin-resistant colonies were collected and cell cloning was done by limited dilution method. Individual cell clones were solubilized to confirm the expression of the full-length human GPC3 by western blotting using the anti-GPC3 antibody. A cell strain stably expressing human GPC3 was obtained.

Example 6

ADCC assay using PBMC derived from human peripheral blood

(1) Preparation of human PBMC

Peripheral blood was collected from normal subjects with heparinized syringes, and diluted to 2 fold with PBS (-), and overlaid on Ficoll-Paque™ PLUS (Amersham Pharmacia Biotech AB) . This was centrifuged (500 × g, 30 minutes, 20 °C), and collected the intermediate layer as a mononuclear cell fraction. After rinsing three times, the resulting fraction was suspended in 10 % FBS/RPMI to prepare a human PBMC solution.

(2) Preparation of target cell

HepG2 cell cultured in 10 % FBS/RPMI 1640 culture medium was detached from the dish using trypsin-EDTA (Invitrogen Corp), divided in each well at $1 \times 10^4$ cells/well in a U-bottom 96-well plate (Falcon), and cultured for 2 days. After culturing, 5.55 MBq of chromium-51 was added and the cells were incubated in a 5 % $CO_2$ gas incubator at 37 °C for one hour. The resulting cells were rinsed once with the culture medium, to which 50 μl of 10 % FBS/RPMI 1640 culture medium was added to prepare a target cell.

(3) Chromium release test (ADCC activity)

50 μl of an antibody solution prepared to each concentration was added to the target cell on ice for 15 minutes. Subsequently, 100 μl of a human PBMC solution was added ($5 \times 10^5$ cells/well), and incubated in a 5 % $CO_2$ gas incubator at 37 °C for 4 hours. After incubation, the plate was centrifuged and the radioactivity in 100 μl of the culture supernatant was counted with a gamma counter. The specific chromium release ratio was determined by the following formula:

$$\text{Specific chromium release ratio (\%)} = (A-C) \times 100/(B-C)$$

"A" represents the mean radioactivity value (cpm) in each well; "B" represents the mean radioactivity value (cpm) in a well where 100 μl of aqueous 2 % NP-40 solution (Nonidet P-40, Code No. 252-23, Nakarai Tesque) and 50 μl of 10 % FBS/RPMI culture medium were added to the target cell; and "C" represents the mean radioactivity value (cpm) in a well where 150 μl of 10 % FBS/RPMI culture medium was added to the target cell. The test was done in triplicate to calculate the mean of the ADCC activity (%) and the standard error.

The results are shown in Fig.10. Among the six types of anti-GPC3 chimera antibodies, the antibodies ch.M3C11 and ch.M1E07 recognizing the C terminus exerted the ADCC activity, while the antibodies ch. M19B11, ch. M18D04, ch. M5E09 and ch. M10D02 recognizing the N terminus hardly exerted the ADCC activity. The above results indicate that the ADCC activities of the chimera antibodies depend on the recognition sites of the antibodies. Further, it was expected that the antibodies recognizing the C terminus of GPC3 were possibly useful in clinical applications since the antibodies recognizing the C terminal sides from the cleavage sites exerted the ADCC activity.

Example 7

Assay of compliment-dependent cytotoxic activity (CDC activity)

(1) Preparation of human albumin veronal buffer (HAVB)

12.75 g of NaCl (superior grade; Wako Pure Chemical Industries, Ltd.), 0.5625 g of Na-barbital (superior grade; Wako Pure Chemical Industries, Ltd.), and 0.8625 g of barbital (superior grade; Wako Pure Chemical Industries, Ltd.) were dissolved in Milli Q water to 200 mL, and autoclaved (121 °C, 20 minutes) . 100 mL of autoclaved warm Milli Q water was added. Then, it was confirmed that the resulting mixture was at pH 7.43 (pH 7.5 recommended). This was defined as 5 × Veronal Buffer. 0.2205 g of $CaCl_2$-$2H_2O$ (superior grade; Wako Pure Chemical Industries, Ltd.) was dissolved in 50 mL of Milli Q water to 0.03 mol/L. The resulting solution was defined as $CaCl_2$ solution. 1.0165 g of $MgCl_2$-6 $H_2O$ (superior grade; Wako Pure Chemical Industries, Ltd.) was dissolved in 50 mL of Milli Q water to 0.1 mol/L. The resulting solution was defined as $MgCl_2$ solution. 100 mL of 5 × Veronal Buffer, 4 mL of human serum albumin (Buminate$^R$ 25 %, 250 mg/mL of human serum albumin concentration, Baxter), 2.5 mL of the $CaCl_2$ solution, 2.5 mL of the $MgCl_2$ solution, 0.1 g of KCl (superior grade; Wako Pure Chemical Industries, Ltd..), and 0.5g of glucose (D (+) -glucose, anhydrous glucose, superior grade; Wako Pure Chemical Industries, Ltd.) were dissolved in Milli Q water to 500 mL. This was defined as HAVB. After filtration and sterilization, the resulting solution was stored at a set temperature of 5 °C.

(2) Preparation of target cell

CHO cell expressing GPC3 on the cell membrane as prepared in Example 4 was cultured in alpha-MEM nucleic acid (+) culture medium (GIBCO) supplemented with 10 % FBS and 0.5 mg/mL Geneticin (GIBCO), detached from the dish using a cell dissociation buffer (Invitrogen Corp), and divided at 1 × $10^4$ cells/well in each well of a 96-well flat bottom plate (Falcon), for culturing for 3 days. After culturing, 5.55 MBq of chromium-51 was added, and incubated in a 5 % $CO_2$ gas incubator at 37 °C for one hour. The resulting cell was rinsed twice with HAVB, to which 50 μl of HAVB was added to prepare a target cell.

(3) Chromium release test (CDC activity)

Each chimera antibody was diluted with HAVB to prepare an antibody solution of 40 μg/mL. The antibody solution was added in a 50 μl-portion to the target cell, which was then left on ice for 15 minutes. Subsequently, baby rabbit compliment (Cedarlane) diluted with HAVB was added in 100 μl portions to each well to a final concentration of 30 % (final antibody concentration of 10 μg/mL), and incubated in a 5 % $CO_2$ gas incubator at 37 °C for 90 minutes. After centrifugation of the plate, a 100-μl portion of the supernatant was recovered from each well, and the radioactivity was measured with a gamma counter. The specific chromium release ratio was determined by the following formula:

$$\text{Specific chromium release ratio (\%)} = (A-C) \times 100/(B-C)$$

"A" represents the mean radioactivity value (cpm) in each well; "B" represents the mean radioactivity value (cpm) in a ' well where 100 gl of aqueous 2 % NP-40 solution (Nonidet P-40, Code No. 252-23, Nakarai Tesque) and 50 μl of HAVB were added to the target cell; and "C" represents the mean radioactivity value (cpm) in a well where 150 μl of HAVB was added to the target cell. The test was done in triplicate to calculate the mean of the CDC activity (%) and the standard error.

The results are shown in Fig.11. Among the six types of the anti-GPC3 chimera antibodies, the antibodies ch.M3C11 and M1E07 recognizing the C terminus exerted the CDC activity, while the antibodies ch. M19B11, ch. M18D04, ch. M5E09 and ch. M10D02 recognizing the N terminus exerted low CDC activities. The above results indicate that the CDC activities of the chimera antibodies depend on the recognition sites of the antibodies. Further, it was expected that the antibodies recognizing the C terminus of GPC3 were possibly useful in clinical applications since the antibodies recognizing the C terminal sides from the cleavage sites exerted the CDC activity.

Industrial Applicability

As shown in the Examples, it was suggested such that a portion of GPC3 highly expressed in hepatoma cells may exist as a secreted form in blood. Because the gene expression of GPC3 is observed at an earlier stage than that of AFP, a hepatoma marker, GPC3 detection is expected to be useful for cancer diagnosis. It is observed that GPC3 is expressed in cancer cell lines other than hepatoma cell lines, such as lung cancer, colon cancer, breast cancer, prostate cancer,pancreatic cancer and lymphoma. Accordingly, GPC3 is possibly applicable to the diagnosis of cancers other than hepatoma.

Additionally, it is also suggested that a secreted form of GPC3 in blood predominantly comprises the N-terminal fragment of about 40 kDa, which is observed in the soluble form of the GPC3 core protein. This indicates that antibodies recognizing the N-terminal fragment are useful as the antibody for use in such diagnosis. In addition, if antibodies recognizing the C-terminal fragment with the ADCC activity and/or the CDC activity are used for treating hepatoma, the antibodies can efficiently reach hepatoma cell without being trapped by the secreted form of GPC3 present in blood. Thus, such antibodies are useful as agents for disrupting cancer cells and as anti-cancer agents.

The contents of all the publications listed in this specification are entirely included in the specification. Additionally, a person skilled in the art will readily understand that various modifications and variations of the invention are possible without departure from the technical scope and inventive range described in the attached claims. It is intended that the invention also encompasses such modifications and variations.

## Claims

1. An antibody against an N-terminal peptide of GPC 3.

2. The antibody claimed in Claim 1 wherein the N-terminal peptide of GPC 3 is a secreted form of a peptide found in blood.

3. The antibody claimed in Claim 2 wherein the N-terminal peptide of GPC 3 is a peptide comprising amino acid residues 1-374 of GPC 3 or a peptide comprising amino acid residues 1-358 of GPC 3.

4. The antibody claimed in Claim 3 wherein the N-terminal peptide of GPC 3 is a peptide comprising amino acid residues 1-358 of GPC 3.

5. The antibody claimed in any one of Claims 1-4 which is a monoclonal antibody.

6. The antibody claimed in Claim 1 which is immobilized to an insoluble support.

7. The antibody claimed in Claim 1 which is labeled with a labeling material.

8. An antibody against a C-terminal peptide of GPC 3.

9. The antibody claimed in Claim 8 wherein the C-terminal peptide of GPC 3 is a peptide comprising amino acid residues 359-580 of GPC 3 or a peptide comprising amino acid residues 375-580 of GPC 3.

10. The antibody claimed in Claims 8 or 9 which is a monoclonal antibody, or a chimera antibody, or a cytotoxic antibody.

Fig. 1

A.

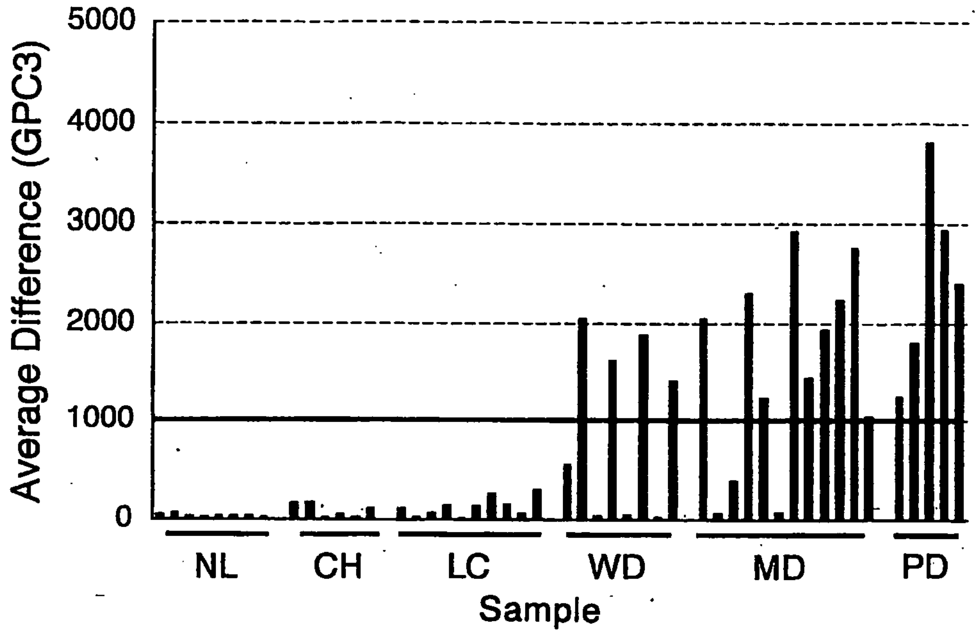
5000
4000
3000
2000
1000
0
Average Difference (GPC3)
NL
CH
LC
WD
MD
PD
Sample

B.

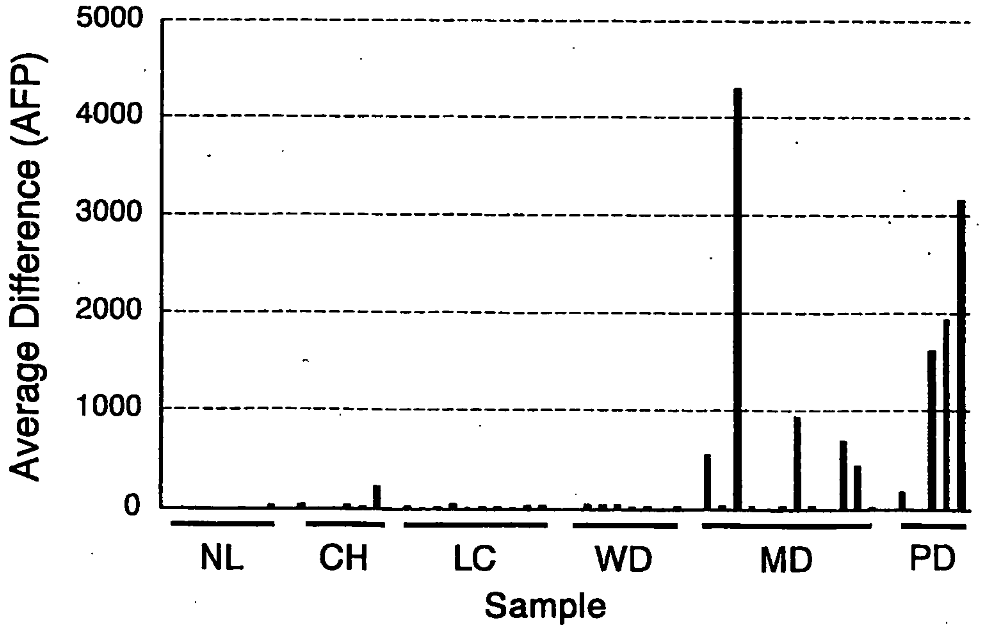
5000
4000
3000
2000
1000
0
Average Difference (AFP)
NL
CH
LC
WD
MD
PD
Sample

Fig. 2

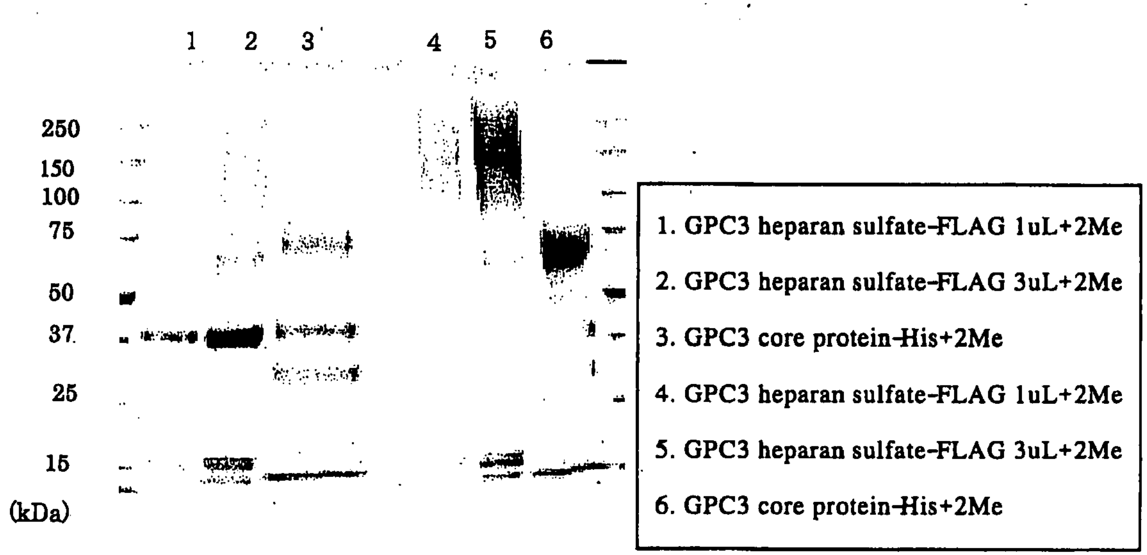
1 2 3 4 5 6
250
150
100
75
50
37
25
15
(kDa)
1. GPC3 heparan sulfate-FLAG 1uL+2Me
2. GPC3 heparan sulfate-FLAG 3uL+2Me
3. GPC3 core protein-His+2Me
4. GPC3 heparan sulfate-FLAG 1uL+2Me
5. GPC3 heparan sulfate-FLAG 3uL+2Me
6. GPC3 core protein-His+2Me

Fig. 3

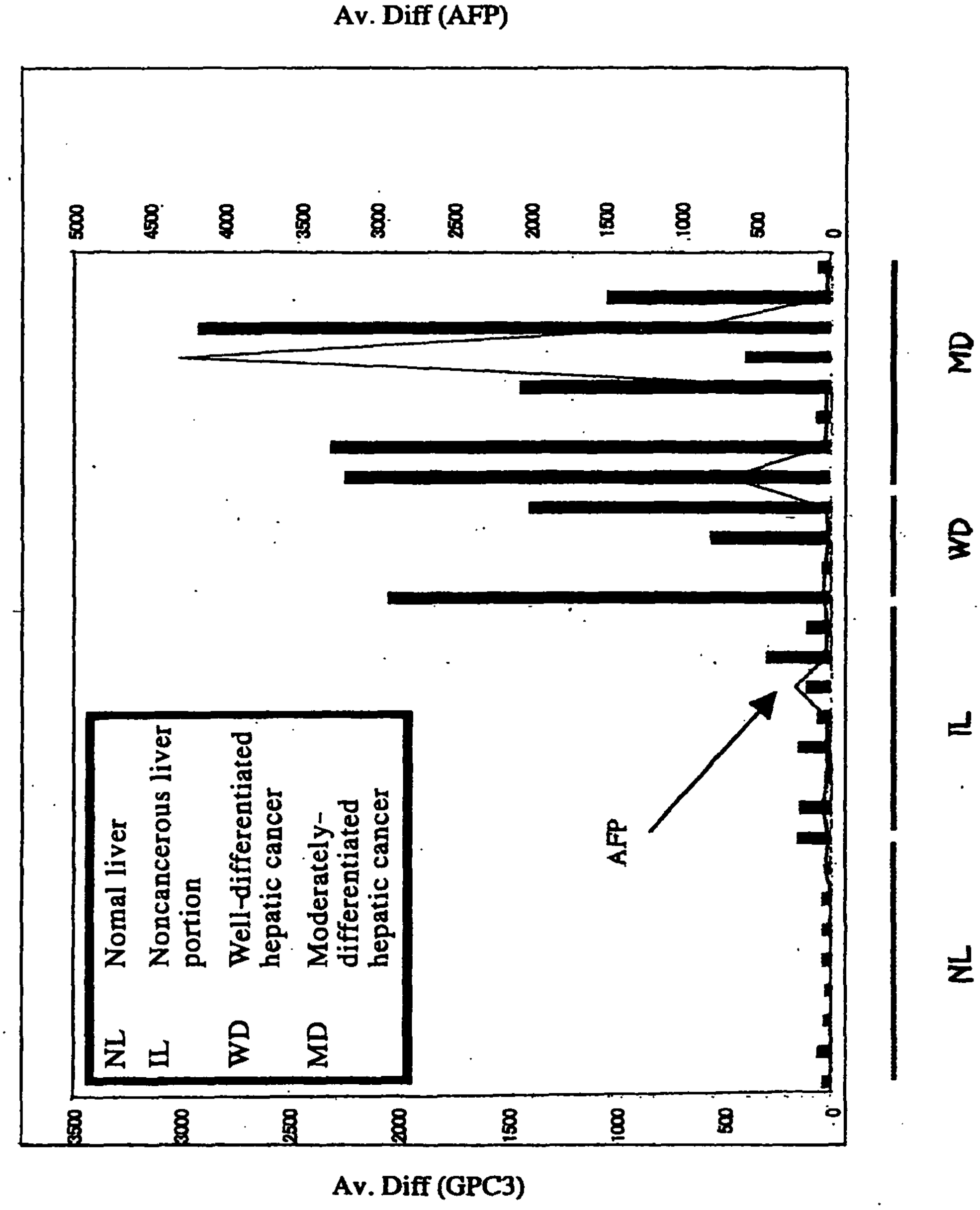
Av. Diff (AFP)
Av. Diff (GPC3)
NL Nomal liver
IL Noncancerous liver portion
WD Well-differentiated hepatic cancer
MD Moderately-differentiated hepatic cancer
AFP
NL
IL
WD
MD
0
500
1000
1500
2000
2500
3000
3500
4000
4500
5000

Fig. 4

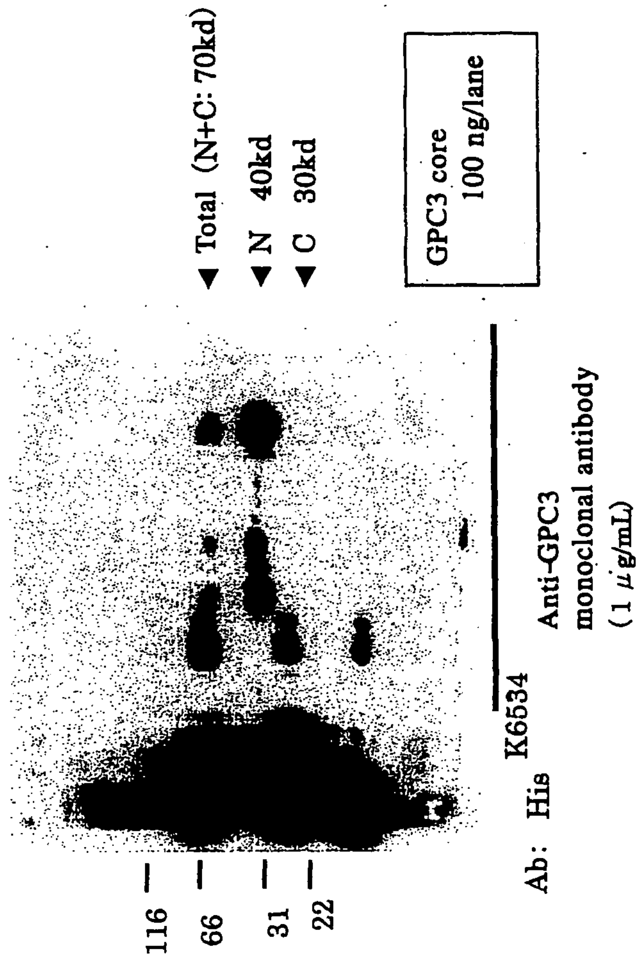

Total (N+C: 70kd)
N 40kd
C 30kd
GPC3 core
100 ng/lane
116
66
31
22
Ab: His
K6534
Anti-GPC3
monoclonal antibody
(1 μg/mL)

Fig. 5

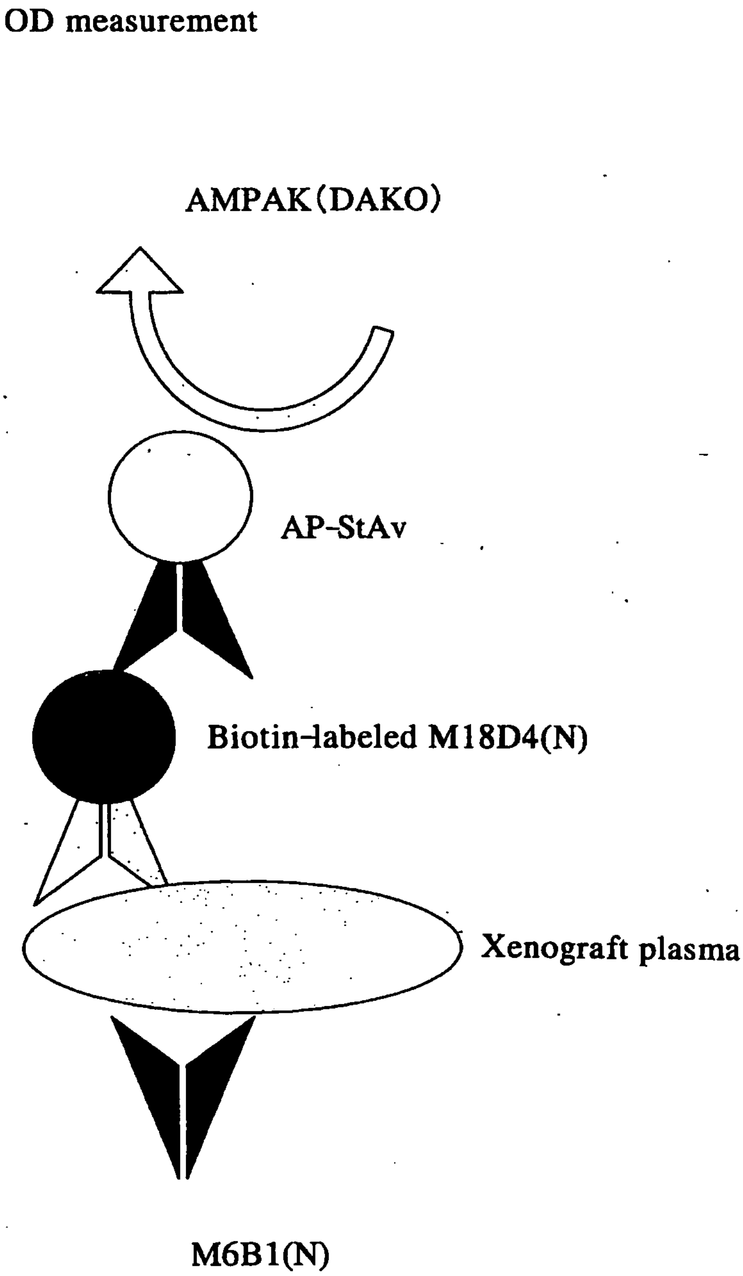
OD measurement
AMPAK(DAKO)
AP-StAv
Biotin-labeled M18D4(N)
Xenograft plasma
M6B1(N)

Fig. 6

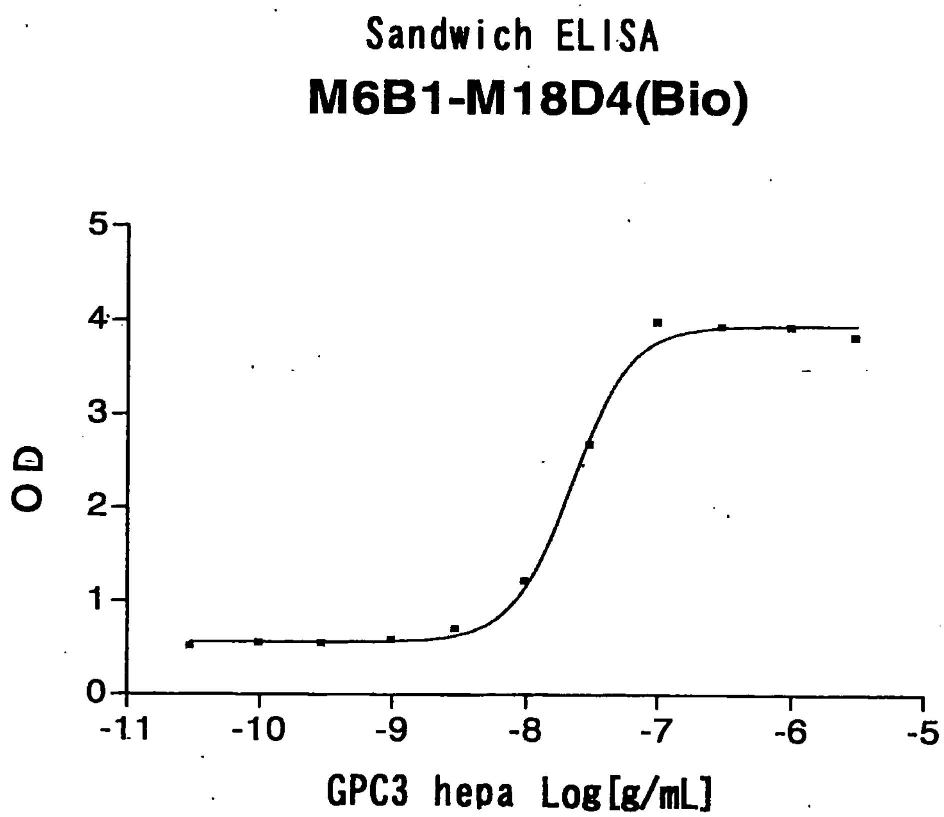
Sandwich ELISA
M6B1-M18D4(Bio)
OD
0
1
2
3
4
5
-11
-10
-9
-8
-7
-6
-5
GPC3 hepa Log[g/mL]

Fig. 7

N-terminal-recognizing antibody

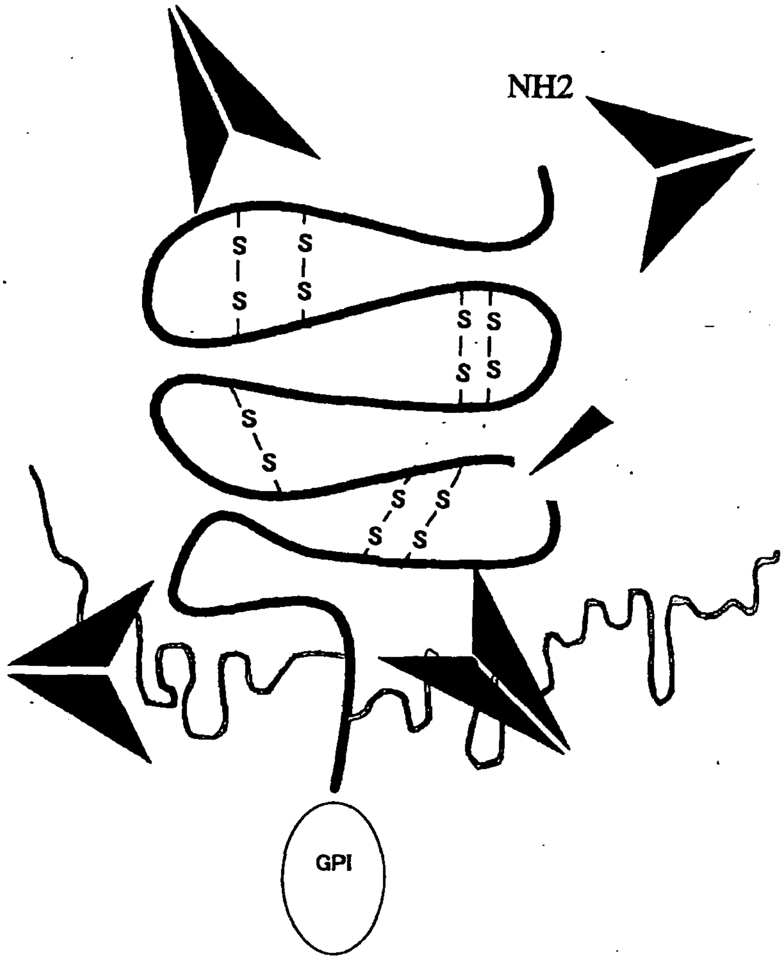


C-terminal-recognizing antibody

Fig. 8

| | Form of soluble GPC3 | | |
|---|---|---|---|
| | N-terminus only | N+C | C-terminus only |
| N-N ELISA | ⊕ | ⊕ | — |
| N-C ELISA | — | ⊕ | — |
| C-C ELISA | — | ⊕ | ⊕ |

Fig. 9

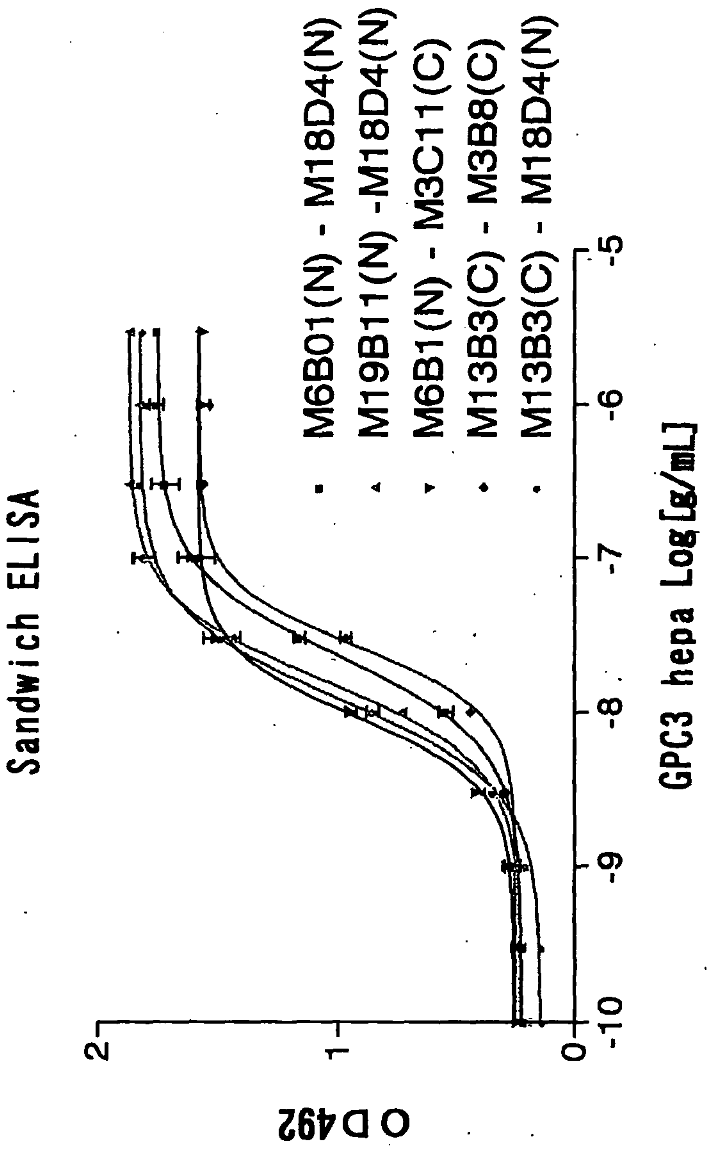
Sandwich ELISA
O D492
GPC3 hepa Log[g/mL]
M6B01(N) - M18D4(N)
M19B11(N) -M18D4(N)
M6B1(N) - M3C11(C)
M13B3(C) - M3B8(C)
M13B3(C) - M18D4(N)

Fig. 10

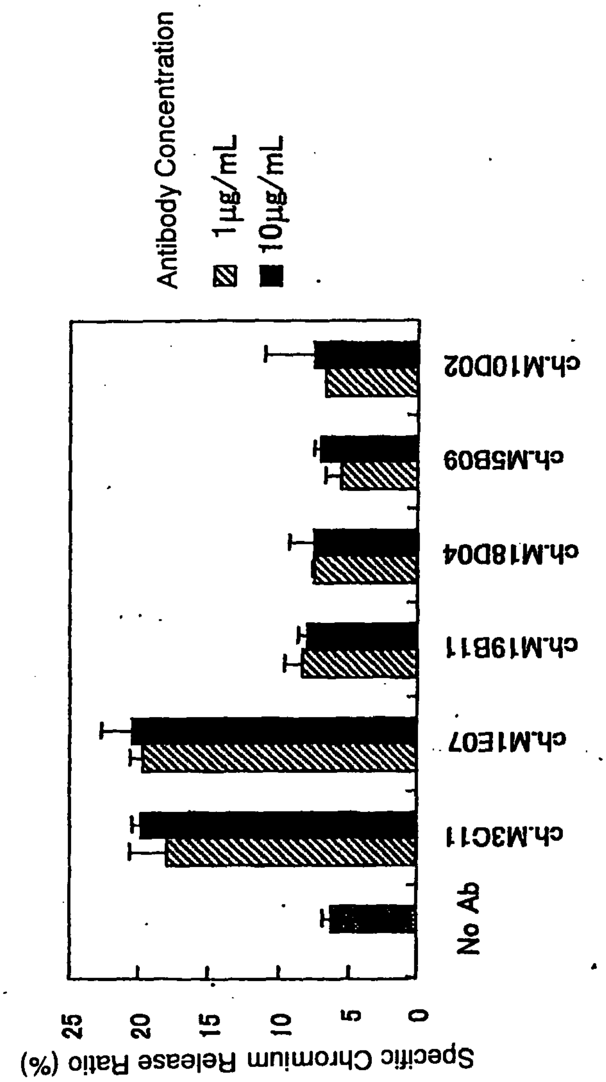
Specific Chromium Release Ratio (%)
25
20
15
10
5
0
No Ab
ch.M3C11
ch.M1E07
ch.M19B11
ch.M18D04
ch.M5B09
ch.M10D02
Antibody Concentration
1μg/mL
10μg/mL

Fig. 11

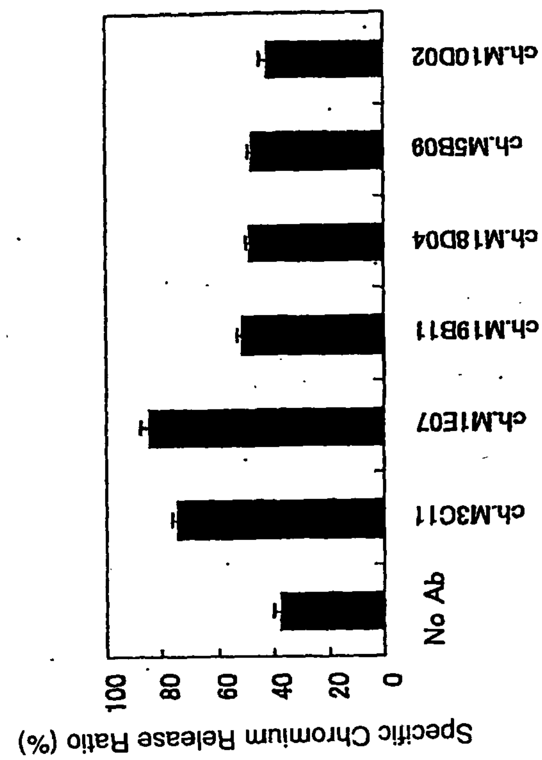
Specific Chromium Release Ratio (%)
100
80
60
40
20
0
No Ab
ch.M3C11
ch.M1E07
ch.M19B11
ch.M18D04
ch.M5B09
ch.M10D02

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001189443 A
- JP 1059878 B
- WO 9425585 A
- WO 9312227 A
- WO 9203918 A
- WO 9402602 A
- WO 9411523 A
- EP 125023 A
- WO 9602576 A
- WO 9813388 A
- WO 0061739 A
- WO 0231140 A
- WO 9219759 A


### Non-patent literature cited in the description


- **LAGE, H. et al.** *Gene,* 1997, vol. 188, 151-156
- *J. Immunol.,* 1979, vol. 123, 1548-1550
- *Current Topics in Microbiology and Immunology,* 1978, vol. 81, 1-7
- **KOHLERG. ; MILSTEIN, C.** *Eur. J. Immunol.,* 1976, vol. 6, 511-519
- **MARGULIES, D. H. et al.** *Cell,* 1976, vol. 8, 405-415
- **SHULMAN, M. et al.** *Nature,* 1978, vol. 276, 269-270
- **DE ST. GROTH, S. F. et al.** *J. Immunol. Methods,* 1980, vol. 35, 1-21
- **TROWBRIDGE, I. S.** *J. Exp. Med.,* 1978, vol. 148, 313-323
- **GALFRE, G. et al.** *Nature,* 1979, vol. 277, 131-133
- **KOHLER G. ; MILSTEIN C.** *Methods Enzymol.,* 1981, vol. 73, 3-46
- **VANDAMME, A. M. et al.** *Eur. J. Biochem.,* 1990, vol. 192, 767-775
- **CHIRGWIN, J. M. et al.** *Biochemistry,* 1979, vol. 18, 5294-5299
- **CHOMCZYNSKI, P. et al.** *Anal. Biochem.,* 1987, vol. 162, 156-159
- **FROHMAN, M.A. et al.** *Proc. Natl. Acad. Sci. USA,* 1988, vol. 85, 8998-9002
- **BELYAVSKY, A. et al.** *Nucleic Acids Res.,* 1989, vol. 17, 2919-2932
- **EBERT, K. M. et al.** *Bio/Technology,* 1994, vol. 12, 699-702
- **SATO, K. et al.** *Cancer Res.,* 1993, vol. 53, 851-856
- **CO, M. S. et al.** *J. Immunol.,* 1994, vol. 152, 2968-2976
- **BETTER, M. ; HORWITZ, A. H.** Methods in Enzymology. Academic Press, Inc, 1989, vol. 178, 476-496
- **PLUECKTHUN, A. ; SKERRA, A.** Methods in Enzymology. Academic Press, Inc, 1989, vol. 178, 476-496
- **LAMOYI, E.** *Methods in Enzymology,* 1989, vol. 121, 652-663
- **ROUSSEAUX, J. et al.** *Methods in Enzymology,* 1989, vol. 121, 663-669
- **BIRD, R. E. et al.** *TIBTECH,* 1991, vol. 9, 132-137
- **HUSTON, J. S. et al.** *Proc. Natl. Acad. Sci. U.S.A.,* 1988, vol. 85, 5879-5883
- **MULLIGAN et al.** *Nature,* 1979, vol. 277, 108
- **WARD et al.** *Nature,* vol. 341, 544-546
- *FASEBJ.,* 1992, vol. 6, 2422-2427
- **BETTER et al.** *Science,* 1988, vol. 240, 1041-1043
- **LEI, S. P. et al.** *J. Bacteriol.,* 1987, vol. 169, 4379
- Antibodies A Laboratory Manual. Cold Spring Harbor Laboratory, 1988
- **HARADA, A. et al.** *International Immunology,* 1993, vol. 5, 681-690
- Remington's Pharmaceutical Science. Mark Publishing Company
- **NIWA et al.** *Gene,* 1991, vol. 108, 193-200
- **SATO et al.** *Mol. Immunol.,* 1994, vol. 31, 371-381